# EUROPEAN PATENT APPLICATION

(11) **EP 4 846 932 A1**
(43) Date of publication of application: **02.12.2026**
(21) Application number: 26179246.9
(22) Date of filing: 18.05.2026
(51) Int. Cl.: A61B 5/022, A61B 5/00

(54) **SYNTHESIZING INFLATE AND DEFLATE WAVEFORMS FOR BLOOD PRESSURE MEASUREMENTS**

(30) Priority: 29.05.2025 US 202563814298 P; 11.05.2026 US 202619673341
(71) Applicant: Stryker Corporation, Portage, MI 49002 (US)
(72) Inventor: MCLOUTH, Justin, Portage, 49002-9711 (US); FURMAN, Aaron, Portage, 49002-9711 (US); LEE, Frank Jian, Portage, 49002-9711 (US); SCHAAF, Darren, Portage, 49002-9711 (US)
(74) Representative: Kilburn & Strode LLP

(57) **Abstract**

An example method includes receiving data indicating an inflation pressure in a cuff inflating around a limb of a subject during an inflation time; receiving data indicating a deflation pressure in the cuff deflating around the limb of the subject during a deflation time; and generating a combined waveform by combining at least a portion of the data indicating the inflation pressure and at least a portion of the data indicating the deflation pressure. The example method further includes determining a systolic blood pressure of the subject by analyzing the combined waveform; determining a diastolic blood pressure of the subject by analyzing the combined waveform; and determining a mean arterial pressure of the subject by analyzing the combined waveform. In addition, the example method includes causing the output device to output indications of the systolic, diastolic and mean arterial blood pressures.

## Description

### BACKGROUND

Blood pressure is an important vital sign that is indicative of a patient's health. Clinically, blood pressure often refers to both a systolic pressure (SBP), which refers to the maximum pressure measured during the cardiac cycle (e.g., one heartbeat), as well as a diastolic pressure (DBP), which refers to a minimum pressure between multiple cardiac cycles. Other clinically relevant blood pressure metrics include mean arterial pressure (MAP) and pulse pressure (PP). Both MAP and pulse pressure are related to systolic and diastolic blood pressure.

Blood pressure can be invasively measured using a catheter inserted into the patient's circulatory system. It can also be measured noninvasively by inflating a blood pressure cuff. The blood pressure cuff may be inflated in order to squeeze an extremity of the patient. Typically, during deflation of the blood pressure cuff the pressures are observed at which systolic and/or diastolic pressures are reached.

### BRIEF DESCRIPTION OF THE DRAWINGS

FIG. 1 illustrates an example environment for determining blood pressure of a subject through synthesis of inflation and deflation waveforms.
FIG. 2 illustrates an example process to generate a combined oscillometric waveform based on cuff pressure data.
FIG. 3 illustrates an example display with a blood pressure measurement mode selection user interface (UI).
FIG. 4A illustrates an example pressure waveform that can be generated using various techniques described herein.
FIG. 4B illustrates an example cuff pressure curve, a section of the cuff pressure curve bounded by Korotkoff sounds, an oscillometric waveform that can be extracted from the cuff pressure curve and optionally gated according to the techniques described herein, and an oscillometric waveform envelope that can be constructed from the oscillometric waveform.
FIG. 5 illustrates an example process for using a combination of inflation and deflation data to noninvasively determine the blood pressure of a subject.
FIG. 6 illustrates another example process for using a combination of inflation and deflation data to noninvasively determine the blood pressure of a subject.
FIG. 7 illustrates an example of an external defibrillator configured to perform various functions described herein.

### DETAILED DESCRIPTION

Existing techniques for blood pressure detection have a number of drawbacks. For example, invasive blood pressure measurements are less practical and more dangerous in out-of-hospital environments. Invasive blood pressure measurements also carry inherent risks for patients, even in controlled clinical environments, such as hospitals.

Further, noninvasive cuff-based blood pressure measurements are prone to error, particularly when the patient, or the cuff, is in motion. Error can lead to repeated measurement attempts, which are uncomfortable for patients. In some cases, a successful and accurate measurement may be impossible.

Various implementations described herein relate to improved techniques for noninvasively detecting the blood pressure of a subject by combining measurement data collected during cuff inflation and deflation. Blood pressure data can be collected during an inflation stage of a blood pressure cuff, and the collected inflation-stage data can be processed to generate an inflation-stage oscillometric waveform. Similarly, blood pressure data can be collected during a deflation stage of a blood pressure cuff, and the collected deflation-stage data can be processed to generate a deflation-stage oscillometric waveform. The inflation-stage oscillometric waveform and the deflation-stage oscillometric waveform can be combined into a combined oscillometric waveform using the techniques described herein. The combined oscillometric waveform can be used to determine various blood pressure measurements, such as a subject's systolic blood pressure, diastolic blood pressure, and/or mean arterial pressure (MAP).

Automated non-invasive blood pressure (NIBP) measurements conducted without the benefit of the techniques described herein can be unreliable and/or inaccurate, especially in uncontrolled conditions such as experienced by first responders administering emergency care. One driver for poor quality measurements in emergency care environments is the susceptibility of NIBP modules to noise and vibration entering a blood pressure cuff and hose during measurements.

Many NIBP modules utilize a "measure on deflate" approach, which involves quickly inflating a cuff to a pressure above a patient's systolic blood pressure, and then deflating in steps or a slow linear ramp, measuring the amplitude of pulse disturbances across the deflation stage. Systolic, diastolic, and MAP pressures are then calculated using a curve formed by the pulse amplitudes across the cuff pressure range, known as the oscillometric waveform and envelope.

Some NIBP modules utilize a "measure on inflate" technique which relies on the same principle as measure on deflate except that pulse amplitudes are collected during inflation of the cuff. A benefit to this approach is an overall faster measurement time because the cuff can be immediately dump deflated once inflation is complete. A downside of the approach, however, is the complexity and challenge of extracting the pulses from the pressure signal while a pump is actively inflating the cuff, which introduces noise.

While some NIBP modules may support measure on inflate or measure on deflate, none currently include the capability to synthesize data from measure on inflate and measure on deflate, as described in this disclosure. Various implementations of the present disclosure can utilize a combination of both measure on inflate and measure on deflate techniques to provide a more reliable, accurate, and in some cases faster blood pressure measurement for the emergency care environment.

An issue that can cause both inaccurate and/or no reading at all from an automated NIBP monitor is the corruption of the pulses in the oscillometric waveform from external noise. In the emergency care environment, the sources of noise that could corrupt/disturb a NIBP measurement are numerous, a few notable sources being vibration or large bumps while in an ambulance and body movements of unstable patients.

This disclosure provides an oscillometric NIBP process that employs both measure on deflate and inflate techniques together, collecting oscillometric waveforms from both techniques and then using data analysis to synthesize them into a combined waveform envelope for calculation of systolic, diastolic, and MAP blood pressure values.

By utilizing data from both the inflation stage and the deflation stage, implementations of this disclosure can significantly increase confidence that collected pulses are true pulses and not artifacts, as it is unlikely that identical noise would occur at the same cuff pressure on both inflate and deflate. Another benefit of solutions according to this disclosure is that if a block of pulses is corrupted during either the deflation stage or the inflation stage, the pulse data from the other stage of the inflation-deflation cycle can be used to fill in the corrupted block, enabling generation of the combined waveform and calculation of blood pressure values. In contrast, a NIBP monitor using only measure on inflate or measure on deflate is likely to either give an inaccurate measurement or no measurement at all if a block of pulses is corrupted, which can be frustrating for emergency responders.

This disclosure enables various useful additional features of blood pressure measurement equipment. In addition to a feature that calculates blood pressure measurements based on combined inflate and deflate data, features such as a selectable measurement speed mode are enabled. One example measurement speed mode can process data from the inflation stage before deflating and can then deflate selectively to those few pressure levels where pulses were missed or corrupted on inflate. This measurement speed mode can optionally be selected by users in view of their use case/situation.

Implementations of the present disclosure will now be described with reference to the accompanying figures.

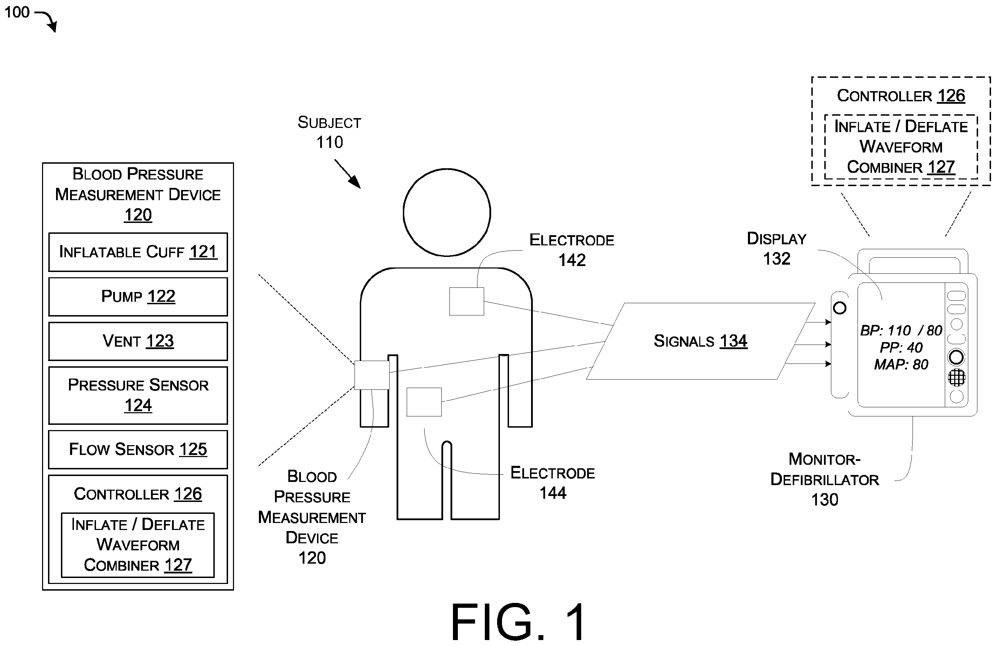

FIG. 1 illustrates an example environment 100 for determining blood pressure of a subject 110 through synthesis of inflation and deflation waveforms. In some examples, the subject 110 is a patient in a clinical environment such as a hospital, clinic, or ambulance. In some cases, the subject 110 is a patient in a non-clinical environment, such as a rescue scene. For instance, the subject 110 may have experienced a medical emergency (e.g., cardiac event, loss-of-consciousness, acute injury, etc.) in an out-of-hospital environment, such as in a school, a workplace, an airport terminal, or a public space. In some cases, the subject 110 is in a home environment. In various examples, it may be beneficial to assess the blood pressure of the subject 110.

FIG. 1 further illustrates a blood pressure measurement device 120, a monitor-defibrillator 130, and electrodes 142, 144. The blood pressure measurement device 120 can include, e.g., an inflatable cuff type blood pressure measurement device. The blood pressure measurement device 120 can include an inflatable cuff 121, a pump 122, a vent 123, a pressure sensor 124, a flow sensor 125, and a controller 126. The controller 126 can include, *inter alia*, an inflate / deflate waveform combiner 127.

The monitor-defibrillator 130 can include, e.g., a display 132 adapted to present measurements, including blood pressure measurements, pertaining to the subject 110. The monitor-defibrillator 130 can also include computing hardware and software such as a processor and memory, which are not illustrated in FIG. 1 for simplicity of illustration. In some embodiments, the controller 126 and inflate / deflate waveform combiner 127, or portions thereof, can optionally be implemented at the monitor-defibrillator 130. The monitor-defibrillator 130 can be adapted to connect to, or may include, external sensors and devices, such as the blood pressure measurement device 120 and the electrodes 142, 144. The monitor-defibrillator 130 can receive signals 134 from external sensors and devices and can process the signals and present resulting data, e.g., the blood pressure data, on the display 132.

In an example according to FIG. 1, the blood pressure measurement device 120 can be installed on the limb of the subject 110, e.g., by wrapping the inflatable cuff 121 around the limb of the subject 110. The blood pressure measurement device 120 can optionally be communicatively coupled with the monitor-defibrillator 130, e.g., via a wired or wireless connection. A blood pressure measurement can be initiated by the subject 110 or by an operator, e.g., an emergency responder, nurse, doctor, or other medical service provider.

In response to a blood pressure measurement initiation, the controller 126 can activate the pump 122 to inflate the inflatable cuff 121. As the inflatable cuff 121 is inflating, the controller 126 can sample and record pressure data via the pressure sensor 124, and the controller 126 can also optionally sample and record blood flow data via the flow sensor 125. The pressures at which blood flow changes occur can indicate various blood pressure parameters, such as systolic and diastolic blood pressures, as understood in the field of blood pressure measurement.

The controller 126 can use the pressure data recorded during the inflation stage, optionally along with the flow data recorded during the inflation stage, to generate a first oscillometric waveform. The waveform, which may be based on the pressure within the cuff over time, oscillates as a result of changes in blood flow (and blood pressure) through the limb of the subject 110 over time. Depending on the amount of noise in the environment 100 and/or other factors affecting the measurement, the first oscillometric waveform may have certain corrupt portions, outlier data points, or missing sections. The controller 126 can optionally identify such portions of the first oscillometric waveform.

After a peak pressure is reached, the controller 126 can deactivate the pump 122, and the controller 126 can activate the vent 123 to deflate the inflatable cuff 121. The controller 126 can optionally operate the vent 123 intermittently to reduce the pressure in the inflatable cuff 121 to selected pressure levels. The controller 126 may be configured to pause at each selected pressure level for a period of time, e.g., a duration of two blood pulses through the limb from two heartbeats of the subject 110. As the inflatable cuff 121 is at each selected pressure level in the deflation stage, the controller 126 can sample and record pressure data via the pressure sensor 124, and the controller 126 can also sample and record blood flow data via the flow sensor 125. As noted herein, the pressures at which blood flow changes occur can indicate systolic and diastolic blood pressures, as well as other blood pressure parameters, such as MAP and pulse pressure.

The controller 126 can use the pressure data recorded during the deflation stage, optionally along with the flow data recorded during the deflation stage, to generate a second oscillometric waveform. An oscillometric waveform is a graphical representation of the amplitude of pressure oscillations (cuff volumetric and pressure change) in an occluding cuff. It is generated by extracting a high frequency (typically 0.5 - 10Hz) pulsatile signal from continuous cuff pressure data collected over time as the cuff inflates and deflates. Depending on the amount of noise in the environment 100 and/or other factors affecting the measurement, the second oscillometric waveform may have certain corrupt portions, outlier data points, or missing sections. The controller 126 can optionally identify such corrupted and/or missing portions of the second oscillometric waveform.

The inflate / deflate waveform combiner 127 can be configured to combine the first oscillometric waveform, which is based on data collected during the inflation stage, with the second oscillometric waveform, which is based on data collected during the deflation stage. The inflate / deflate waveform combiner 127 can optionally use any of a variety of data synthesis techniques, and this disclosure is not limited to any specific technique. Example techniques include averaging, cross-correlating, cross-referencing, gating or filtering, cross-checking, superimposing, interpolating or convolving data indicative of inflation pressures with data indicative of deflation pressures. Some further example techniques include superposition and mathematical curve fitting. Superposition forms a final waveform by adding two waveforms together. Mathematical curve fitting produces a final waveform by minimizing the deviation from both waveforms.

In general, because the first oscillometric waveform is based on data collected during inflation from low pressure to high pressure, while the second oscillometric waveform is based on data collected during deflation from high pressure to low pressure, the first and second oscillometric waveforms can include shapes that represent imperfect mirror-images of each other. The inflate / deflate waveform combiner 127 can be configured to initially flip, rotate, or reverse one of the oscillometric waveforms, either the first oscillometric waveform or the second oscillometric waveform, in order to initially align the first and second oscillometric waveforms so that further waveform combination processing can be applied.

In some examples, the inflate / deflate waveform combiner 127 can be configured to calculate each data point of a combined oscillometric waveform as a combination value which is based on both the first and second oscillometric waveforms. For example, data points on the combined oscillometric waveform can include average amplitude values or any other mathematical combination based on corresponding data points on the first and second oscillometric waveforms.

In various implementations, the inflate / deflate waveform combiner 127 can be configured to identify outlier data points in the first and second oscillometric waveforms. Outlier data points can be those datapoints which are determined to be beyond a threshold distance from other surrounding data points. For example, if amplitude values on a waveform are 1, 1.1, 1.2, 1.4, 2.4, 1.5, 1.7, ..., and the threshold distance for determining outliers is 0.5, then the 2.4 value may be considered an outlier. The inflate / deflate waveform combiner 127 can be configured to remove outlier data points and calculate the data points on the combined oscillometric waveform without using the outlier data points, thereby avoiding error introduction.

In another example technique to combine or synthesize inflation and deflation waveform data, the inflate / deflate waveform combiner 127 can be configured to use one or more features of the first oscillometric waveform (the inflation waveform) to filter or gate the second oscillometric waveform (the deflation waveform). For example, a pulse timing can be determined based on the first oscillometric waveform, and the pulse timing can be then be used to gate or filter the second oscillometric waveform, for example by removing data that falls between pulses from the second oscillometric waveform. In another example, one or more expected pulse amplitudes can be determined based on the first oscillometric waveform, and the pulse amplitudes can be then be used to gate or filter the second oscillometric waveform, for example by removing or modifying, from the second oscillometric waveform, pulse amplitude data that deviates more than a threshold amount from the expected pulse amplitude.

In another example, rhythmic or recurring noise may be identified in the first oscillometric waveform, and the rhythmic or recurring noise can be then be used to gate or filter the second oscillometric waveform, for example by removing rhythmic or recurring noise data from the second oscillometric waveform. In some implementations, rhythmic or recurring noise identified in the first oscillometric waveform can be used to select a preconfigured noise filter that most closely matches the rhythmic or recurring noise. The preconfigured noise filter may include any of a variety of common noise patterns, such as vehicle noise or subject movement noise. The preconfigured noise filter can be then be used to gate or filter the second oscillometric waveform, for example by removing rhythmic or recurring noise data, as defined in the preconfigured noise filter, from the second oscillometric waveform.

In some cases, one or more sections of the first and/or second oscillometric waveforms may be considered to be corrupt or may be missing/unavailable. Corruption may be determined to be present when, for example, the data cannot be fit to a waveform. The inflate / deflate waveform combiner 127 can be configured to use data from the other, non-corrupted waveform (either the first or second oscillometric waveform) to fill in the missing portions of the corrupted waveform. When only one of the oscillometric waveforms includes usable data over a portion of the combined oscillometric waveform, then the inflate / deflate waveform combiner 127 can be configured to rely on the available, uncorrupted oscillometric waveform (either the first or second oscillometric waveform) to construct a section of the combined oscillometric waveform. In the case of missing or corrupted data on both waveforms, implementations can interpolate or fit new data points into a final waveform by predicting the shape and trend of a curve, e.g., as a bell shape.

Furthermore, techniques described herein can optionally include identifying corrupt or missing/unavailable portions of the first oscillometric waveform prior to the deflation stage. The controller 126 can be configured to identify select pressure levels corresponding to the corrupt/missing/unavailable portions of the first oscillometric waveform, and to operate the vent 123 to deflate the inflatable cuff 121 to the selected pressure levels. This can reduce measurement time and corresponding discomfort of the subject 110, by limiting data collection during the deflation stage to those pressure levels that are needed to complete the combined oscillometric waveform.

After the combined oscillometric waveform is generated by the inflate / deflate waveform combiner 127, the combined oscillometric waveform can be relied on for blood pressure measurement determinations such as systolic blood pressure, diastolic blood pressure, and/or MAP. The determined measurements can be output by the controller 126, e.g., by displaying them on a display, or by sending signals 134 representative of the determined measurements to a device such as the monitor-defibrillator 130.

The monitor-defibrillator 130 can be configured to display, via the display 132, blood pressure measurements determined according to the techniques described herein. In some implementations, the monitor-defibrillator 130 can include the controller 126 and the inflate / deflate waveform combiner 127, allowing the monitor-defibrillator 130 to control the other illustrated components of the blood pressure measurement device 120. Other arrangements are also feasible in view of this disclosure, for example, implementing the controller 126 at the blood pressure measurement device 120 while implementing the inflate / deflate waveform combiner 127 at the monitor-defibrillator 130.

The monitor-defibrillator 130 can optionally include numerous other functions in addition to the blood pressure measurement functions described herein. For example, the monitor-defibrillator 130 can receive pulse data from the electrodes 142 and can be configured to control or deliver defibrillation and optionally chest compressions to the subject 110. One example monitor-defibrillator 130 is the LIFEPAK 35^{®} made by STRYKER^{®} Corporation of Portage, Michigan, USA.

The flow sensor 125 can be implemented via any of a variety of sensor types. One example flow sensor 125 can include an acoustic sensor. As used herein, the terms "blood flow," "blood flow parameters," and their equivalents, may refer to one or more physiological parameters indicative of a movement of blood through a blood vessel. For example, the term "blood velocity" may refer a change in position with respect to time (i.e., distance per time, such as meters per second) of one or more blood cells moving in a blood vessel. The term "blood speed" may refer to a magnitude of a velocity (i.e., distance per time) of one or more blood cells moving in a blood vessel. The term "velocity profile," for example, may refer to a velocity of blood in a blood vessel with respect to an area defined within the blood vessel, such as a velocity of blood in a blood vessel with respect to a location along a cross-section of the blood vessel. The term "flow rate," and its equivalents, may refer to a volume or mass of blood that passes a boundary (e.g., a cross-section of a blood vessel) with respect to time. The terms "net flow," "net flow volume," and their equivalents, may refer to a volume or mass of blood that passes a boundary during a discrete time interval, such as during a cardiac cycle. A net flow volume can be calculated by integrating a flow rate over the time interval. Unless otherwise specified explicitly or by context, the term "blood flow" may refer to blood velocity, blood speed, velocity profile, flow rate, net flow, or any other flow-related parameter.

Blood flow periodically changes based on a cardiac cycle of the subject 110. During systole, the heart muscle contracts and propels the blood through vasculature of the subject 110. During diastole, the heart muscle relaxes and fills with the blood. The cardiac cycle of the subject 110 is repeated for each heartbeat. The activation of the heart muscle during the cardiac cycle produces an electrical signal that can be detected via an electrocardiogram (ECG). If the subject 110 does not have an arrhythmia or other medical condition, the cardiac cycle produces cyclical changes in the blood flow and blood pressure with respect to time. Notably, the resultant changes in blood flow and blood pressure may be slightly delayed compared to the actions of the heart of the subject 110, depending on the distance along the vasculature of the subject 110 between the heart and the sensor location.

The blood flow in the subject 110 exhibits a first peak during systole and a second peak during diastole. In various cases, the blood flow has a greater first peak during systole than the second peak during diastole.

According to various implementations, the blood pressure measurement device 120, or another sensor coupled with the monitor-defibrillator 130, can be configured to sample blood flow multiple times during the cardiac cycle of the subject 110. In some cases, the blood pressure measurement device 120 samples the blood flow at one or more sampling rates, such as 5 Hz, 10 Hz, 50 Hz, 100 Hz, 200 Hz, 1 kHz, 2 kHz, 3 kHz, 4 kHz, 5 kHz, 10 kHz, or the like. In some cases, the blood pressure measurement device 120 samples the blood flow during multiple cardiac cycles of the subject 110. According to various cases, the blood pressure measurement device 120 or the monitor-defibrillator 130 can be configured to generate a waveform of the blood flow over time for each cardiac cycle.

In some examples, the blood pressure measurement device 120 or the monitor-defibrillator 130 can perform laser or ultrasound Doppler velocimetry in order to detect the blood velocity. In various cases, the blood pressure measurement device 120 or the monitor-defibrillator 130 can include or be coupled with one or more interferometric sensors. For example, the blood pressure measurement device 120 or another sensor coupled with the monitor-defibrillator 130 can output light beams (e.g., coherent light beams) that are split (e.g., by one or more mirrors) prior to transmission through the skin. The blood pressure measurement device 120 or the monitor-defibrillator 130 may detect the blood velocity in a blood vessel by comparing a return of the first beam and a return of the second beam to the split beams generated from the first incident beam and the second incident beam. In some implementations, the blood pressure measurement device 120 or the monitor-defibrillator 130 includes, or is coupled to, an ultrasound transducer configured to emit an incident ultrasound beam toward the blood vessel and detect a reflection of the ultrasound beam from blood in the blood vessel. In various cases, a difference in phase and/or frequency between the incident ultrasound beam and the reflection of the ultrasound beam is indicative of the velocity of the blood.

In various examples, the blood pressure measurement device 120 or the monitor-defibrillator 130 can be configured to detect a pulse pressure of the subject 110. The term "pulse pressure" may refer to a difference between a systolic blood pressure and a diastolic blood pressure. For instance, the pulse pressure of the subject 110 is dependent on the maximum change of pressure in a blood vessel that the heart of the subject 110 produces each time it transitions from the end of diastole to the peak of systole. In various cases, the pulse pressure can be calculated as a function of a change in the blood flow over the course of the cardiac cycle as well as the pulse wave velocity of a pulse wave. According to some examples, the pulse pressure is dependent on the change of a velocity of the blood between the systolic phase and the diastolic phase of the cardiac cycle.

In various implementations, the blood pressure measurement device 120 or the monitor-defibrillator 130 can be configured to detect a mean arterial pressure (MAP) of the subject 110. MAP may refer to an average blood pressure in a blood vessel during a single cardiac cycle. According to various cases, the blood pressure measurement device 120 can calculate the MAP as a function of the mean of the blood flow through a blood vessel during the cardiac cycle.

In particular cases, the blood pressure measurement device 120 can be configured to detect an instantaneous blood pressure in the subject 110. The instantaneous blood pressure may vary over time during the cardiac cycle of the subject 110, and may be sampled multiple times during the cardiac cycle. For instance, the instantaneous blood pressure may be represented as a time-varying waveform. In some cases, the blood pressure measurement device 120 is configured to detect a systolic blood pressure, which is typically equal to a peak blood pressure during the systolic phase of the cardiac cycle. In various implementations, the blood pressure measurement device 120 detects the systolic blood pressure by identifying a maximum of the blood pressure waveform over the cardiac cycle. In some examples, the blood pressure measurement device 120 is configured to detect a diastolic blood pressure, which is typically equal to a minimum blood pressure during the end of the diastolic phase of the cardiac cycle. In some cases, the blood pressure measurement device 120 the diastolic blood pressure by identifying a minimum of the blood pressure waveform over the cardiac cycle.

In various cases, blood pressure metric(s) can be dependent on the size of the subject 110. In particular implementations, the blood pressure metric(s) can depend on factors such as body fat, musculature, or other soft tissues. Physiologically, the size of the subject 110 is related to the load on the heart of the subject 110 that is provided by the vasculature of the subject 110. Various blood pressure metrics described herein (e.g., pulse pressure, MAP, etc.) are related to the vasculature load of the subject 110. In various examples, the blood pressure measurement device 120 identifies a metric indicative of the size of the subject 110. In some cases, the size is indicated by a height, a weight, a body mass index (BMI), a height-weight ratio (e.g., a height of the subject 110 divided by a weight or mass of the subject 110), a clothing size (e.g., a shirt size, a pants size, a dress size, etc.), a circumference (e.g., of the neck of the subject 110, of the waist of the subject 110, of the wrist of the subject 110, of the limb upon which the cuff is placed, etc.), a volume of at least a portion of the subject 110, or some other indication. In some cases, the size indicates whether the subject 110 is pregnant. In some examples, the blood pressure measurement device 120 or the monitor-defibrillator 130 receives an input signal (e.g., from a user, such as a rescuer) that indicates the size of the subject 110. For instance, the user may measure, estimate, or otherwise identify the size of the subject 110. In some implementations, the blood pressure measurement device 120 or the monitor-defibrillator 130 is communicatively coupled to an electronic medical record (EMR) device which may communicate the size of the subject 110 as indicated in an EMR of the subject 110.

In various implementations of the present disclosure, the blood pressure measurement device 120 identifies one or more blood pressure metrics of the subject 110 based, at least in part, on the size of the subject 110. For instance, the function utilized by the blood pressure measurement device 120 to calculate pulse pressure or MAP may further depend on the metric indicative of the size of the subject 110. In some examples, a look-up table utilized by the blood pressure measurement device 120 to identify the pulse pressure or MAP may further depend on the size of the subject 110.

According to some examples, the blood pressure measurement device 120 and/or the monitor-defibrillator 130 can be configured to output an indication of blood pressure metric(s). In some cases, the blood pressure measurement device 120 and/or the monitor-defibrillator 130 can include a display configured to visually present the blood pressure metric(s). For instance, the blood pressure measurement device 120 and/or the monitor-defibrillator 130 may display waveforms representative of the blood pressure over the course of the cardiac cycle, such as the first oscillometric waveform, the second oscillometric waveform, and/or the combined oscillometric waveform. In some examples, the blood pressure measurement device 120 and/or the monitor-defibrillator 130 can include a speaker configured to audibly report the blood pressure metric(s). In some implementations, the blood pressure measurement device 120 and/or the monitor-defibrillator 130 can output an alert upon determining that the blood pressure metric(s) are below a first threshold or above a second threshold. The alert, for instance, may be visual or audible.

In some cases, the blood pressure measurement device 120 transmits, to an external device, signals 134 indicating blood pressure metric(s). In some cases, the signals 134 are transmitted over a wireless interface. For instance, the signals 134 may be BLUETOOTH^{™} signals. The signals 134, for instance, encode data indicative of blood pressure metric(s). In some cases, the communication signals 134 include multiple transmissions that collectively report ongoing measurements identified by the blood pressure measurement device 120.

In some examples, the blood pressure measurement device 120 transmits the signals 134 to a monitor-defibrillator 130. For instance, the monitor-defibrillator 130 includes a display 132 configured to visually present blood pressure metric(s). In some examples, the monitor-defibrillator 130 calculates blood pressure metric(s) based on reported characteristic(s), in accordance with the techniques described herein.

In some implementations, the blood pressure measurement device 120 can be configured to determine one or more metrics described herein by taking an average (e.g., median, mean, etc.) of multiple measurements. In some cases, the blood pressure measurement device 120 selectively outputs, or reports, a running average of multiple (e.g., five, ten, etc.) measurements acquired over time. By aggregating the measurements in the form of an average, the quality of the metrics output by the blood pressure measurement device 120 may be enhanced.

In various cases, the blood pressure measurement device 120 can be configured to determine a quality metric associated with various metrics or measurements described herein. For example, the blood pressure measurement device 120 can be configured to determine a variance, Z-score, or other statistic indicative of the quality of a given metric or measurement. In some cases, the blood pressure measurement device 120 can refrain from outputting a metric that is associated with a quality metric that is below a predetermined threshold. In some examples, the blood pressure measurement device 120 can output an average of multiple measurements or calculations that are averaged in view of their respective quality metrics. For instance, the blood pressure measurement device 120 may calculate five estimates of pulse pressure, taken over five separate instances of the cardiac cycle. The blood pressure measurement device 120 may determine a Z-score associated with each of the five estimates in view of each other. Further, the blood pressure measurement device 120 may output an average of the estimates of pulse pressure, weighted in view of the respective Z-scores, such that estimates with a lower Z-score are more represented in the average than estimates with a higher Z-score. Various implementations of the present disclosure reduce the impact of noise on the accuracy of reported metrics.

In some implementations, the blood pressure measurement device 120 can further indicate a confidence (e.g., the quality metric) associated with any metric it reports. For example, the blood pressure measurement device 120 may output an indication of the variance of blood pressure metric measurements used to generate an average blood pressure metric, along with the average blood pressure metric, in order to report a reliability of the average blood pressure metric as reported. In some cases, the blood pressure measurement device 120 can display the average blood pressure metric with a color associated with the variance, such that the average blood pressure metric may be displayed in red if it is based off of measurements with a high variance or the average blood pressure metric may be displayed in green if it is based off of measurements with a low variance. In some cases, the confidence associated with the reported metric is numerically reported.

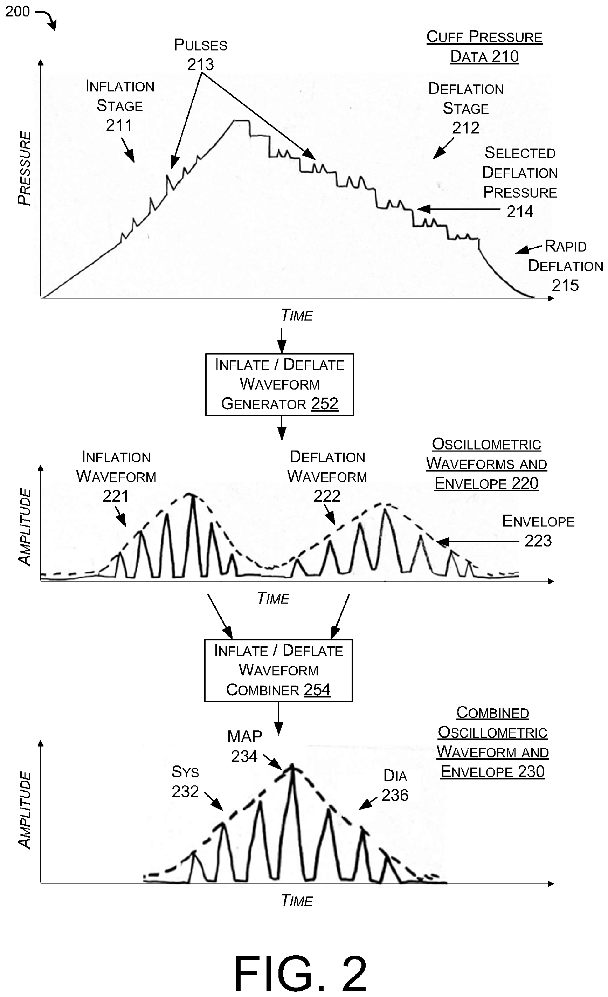

FIG. 2 illustrates an example process 200 to generate a combined oscillometric waveform based on cuff pressure data. The process 200 can be performed, e.g., at a controller 126 such as illustrated in FIG. 1. The process 200 involves cuff pressure data 210, inflate/deflate waveform generator 252, oscillometric waveforms and envelope 220, inflate/deflate waveform combiner 254, and combined oscillometric waveform and envelope 230. The cuff pressure data 210, oscillometric waveforms and envelope 220, and combined oscillometric waveform and envelope 230 can each include data that is collected and/or output by the controller 126. The inflate/deflate waveform generator 252 and the inflate/deflate waveform combiner 254 can include components implemented at the controller 126. For example, the inflate/deflate waveform combiner 254 can implement the inflate/deflate waveform combiner 127 in some embodiments.

The cuff pressure data 210 represents example data collected by a controller 126 from a pressure sensor 124 during inflation and deflation of an inflatable cuff 121. The cuff pressure data 210 includes an inflation stage 211 and a deflation stage 212. The cuff pressure data 210 further includes pulses 213, which manifest as pressure changes in the cuff pressure data 210. The cuff pressure data 210 further includes multiple selected deflation pressures, which manifest as plateaus in the deflation stage 212, such as the example selected deflation pressure 214. The deflation stage 212 also includes a rapid deflation 215 wherein an inflatable cuff is rapidly deflated after sufficient blood pressure measurement data is collected. The inflate/deflate waveform generator 252 can be configured to use the cuff pressure data 210 to generate the oscillometric waveforms and envelope 220.

The oscillometric waveforms and envelope 220 includes an inflation waveform 221, a deflation waveform 222, and an envelope 223 represented by the dotted line. The inflation waveform 221 may also be referred to herein as a first oscillometric waveform, and the deflation waveform 222 may also be referred to herein as a second oscillometric waveform. The inflate/deflate waveform generator 252 can generate the inflation waveform 221 based on the data in the inflation stage 211 of the cuff pressure data 210. The inflate/deflate waveform generator 252 can generate the deflation waveform 222 based on the data in the deflation stage 212 of the cuff pressure data 210. The inflate/deflate waveform generator 252 can generate the envelope 223 based on the inflation waveform 221 and the deflation waveform 222, e.g., by fitting an envelope curve to the peaks of the inflation waveform 221 and the deflation waveform 222. The inflate/deflate waveform combiner 254 can be configured to use the inflation waveform 221 and the deflation waveform 222, optionally along with the envelope 223, to generate the combined oscillometric waveform and envelope 230.

The combined oscillometric waveform and envelope 230 includes an example portion which can be used to derive systolic blood pressure, referred to as Sys 232, an example portion which can be used to derive MAP, referred to as MAP 234, and an example portion which can be used to derive diastolic blood pressure, referred to as Dia 236. The inflate/deflate waveform combiner 254 can be configured to use any of the various techniques described herein to combine the inflation waveform 221 and the deflation waveform 222 to thereby generate the combined oscillometric waveform and envelope 230. A controller 126 can be adapted to derive blood pressure measurements such as systolic blood pressure, MAP, and diastolic blood pressure based on the combined oscillometric waveform and envelope 230.

In an example according to FIG. 2, the cuff pressure data 210 can be collected as described with reference to FIG. 1, namely, by a controller 126 activating the pump 122 to inflate the inflatable cuff 121, and sampling and recording pressure data via the pressure sensor 124 during the inflation stage. The controller 126 can then deactivate the pump 122 and activate the vent 123 to deflate the inflatable cuff 121 and can sample and record pressure data via the pressure sensor 124 during the deflation stage.

After the cuff pressure data 210 is collected, the controller 126 can employ the inflate/deflate waveform generator 252 to generate the inflation waveform 221 and the deflation waveform 222 based on the cuff pressure data 210. The inflation waveform 221 and the deflation waveform 222 can represent pressure changes associated with the pulses 213. The inflation waveform 221 and the deflation waveform 222 can optionally be bounded based at least in part on blood flow data gathered from the flow sensor 125. Depending on the amount of noise in the environment 100 and/or other factors affecting measurement, the inflation waveform 221 and the deflation waveform 222 may contain certain corrupt portions, outlier data points, or missing sections. The inflate/deflate waveform generator 252 can optionally identify such portions of the inflation waveform 221 and the deflation waveform 222.

The inflate / deflate waveform combiner 254 can be configured to combine the inflation waveform 221 with the deflation waveform 222. The inflate / deflate waveform combiner 254 can optionally use any of a variety of data synthesis techniques, and this disclosure is not limited to any specific technique.

The inflate / deflate waveform combiner 254 can be configured to initially flip, rotate, or reverse one of the oscillometric waveforms, either the inflation waveform 221 or the deflation waveform 222, in order to initially align the inflation waveform 221 with the deflation waveform 222 so that further waveform combination processing can be applied. The inflate / deflate waveform combiner 254 can optionally also use the envelope 223 to initially align the inflation waveform 221 with the deflation waveform 222 so that, e.g., peaks and troughs in the inflation waveform 221 generally overlap corresponding peaks and troughs in the deflation waveform 222. The inflate / deflate waveform combiner 254 can be configured to calculate each data point of the combined oscillometric waveform included in the combined oscillometric waveform and envelope 230 as a combination value which is based on both the inflation waveform 221 and the deflation waveform 222. For example, data points on the combined oscillometric waveform can include average values or any other mathematical combination based on corresponding data points on the inflation waveform 221 and the deflation waveform 222.

The inflate / deflate waveform combiner 254 can be configured to remove outlier data points and calculate the data points on the combined oscillometric waveform without using the outlier data points, thereby avoiding error introduction. Furthermore, the inflate / deflate waveform combiner 254 can be configured to identify one or more corrupt or missing sections of the inflation waveform 221 and/or the deflation waveform 222. The inflate / deflate waveform combiner 127 can be configured to use data from the other, non-corrupted waveform (either the inflation waveform 221 or the deflation waveform 222) to fill in the corrupt or missing portions of incomplete waveform. When only one of the inflation waveform 221 or the deflation waveform 222 includes usable data over a portion of the combined oscillometric waveform, then the inflate / deflate waveform combiner 254 can be configured to rely on the available, uncorrupted oscillometric waveform (either the inflation waveform 221 or the deflation waveform 222) to construct a section of the combined oscillometric waveform.

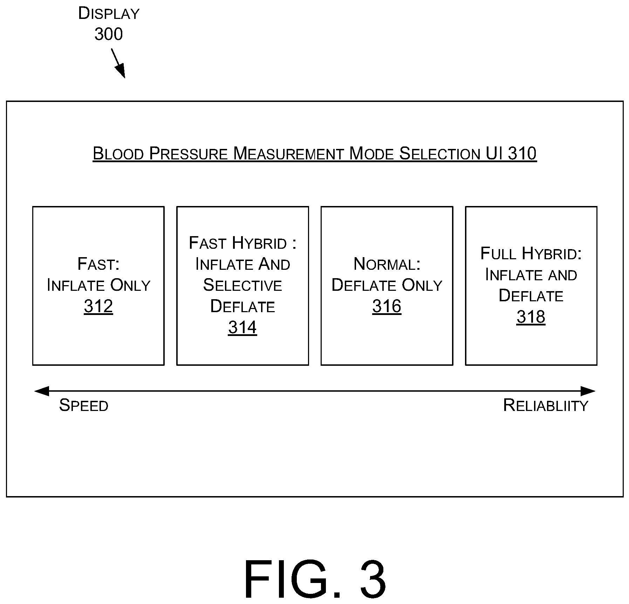

Furthermore, the inflate / deflate waveform combiner 254 can be configured to use one or more features of the inflation waveform 221 to filter or gate the deflation waveform 222, e.g., using pulse timing, pulse amplitude, and/or rhythmic or recurring noise identified in the inflation waveform 221 to filter or gate the deflation waveform 222, as described herein. FIG. 3 illustrates an example display 300 with a blood pressure measurement mode selection user interface (UI) 310. The display 300 can implement, e.g., a display 132 on a monitor-defibrillator 130 such as illustrated in FIG. 1, and mode selections made by the UI 310 can be transmitted to the blood pressure measurement device 120. Alternatively, the display 300 can implement a display at the blood pressure measurement device 120 itself.

The UI 310 includes various example mode selection elements which can be selected, e.g., by a user touching or otherwise selecting the mode selection elements. The example mode selection elements include, "Fast: Inflate Only" 312, "Fast Hybrid: Inflate and Selective Deflate" 314, "Normal: Deflate Only" 316, and "Full Hybrid: Inflate and Deflate" 318. The example mode selection elements are arranged along a speed vs. reliability axis, with "Fast: Inflate Only" 312 at the highest speed/lowest reliability end of the axis, and the other example mode selection elements being arranged in order of descending measurement speed and ascending measurement reliability.

The "Fast: Inflate Only" 312 mode can include a mode in which a blood pressure measurement device, such as the blood pressure measurement device 120 illustrated in FIG. 1, is configured to collect cuff pressure data during the inflate stage only. After the inflate stage, the inflation cuff can be rapidly deflated. The blood pressure measurement device 120 can base any blood pressure measurements such as systolic blood pressure, diastolic blood pressure, and MAP on the data collected during the inflate stage.

The "Fast Hybrid: Inflate and Selective Deflate" 314 mode can include a mode in which a blood pressure measurement device, such as the blood pressure measurement device 120 illustrated in FIG. 1, is configured to collect cuff pressure data during the inflate stage and to determine, based on the inflate stage data, whether any deflate stage data can be collected to correct, enhance, or complete the inflate stage data. If the inflate stage data is complete and sufficiently reliable, then no deflate stage data need be collected. However, if the inflate stage data is corrupt, erratic, or incomplete, then deflate stage data can be determined to be needed.

Furthermore, the blood pressure measurement device 120 can identify select deflation pressures corresponding to missing or corrupted inflation stage data. The blood pressure measurement device 120 can then operate the vent 123 to deflate the inflatable cuff 121 to the select deflation pressures, and the blood pressure measurement device 120 can collect cuff pressure data at the select deflation pressures. The resulting cuff pressure data can be used to generate one or more portions of a deflation waveform, which can be combined with the inflation waveform to generate a combined waveform according to the techniques described herein.

The "Normal: Deflate Only" 316 mode can include a mode in which a blood pressure measurement device, such as the blood pressure measurement device 120 illustrated in FIG. 1, is configured to collect cuff pressure data during the deflate stage only. The blood pressure measurement device 120 can inflate the inflatable cuff 121 without collecting pressure data, and the blood pressure measurement device 120 can then deflate the inflatable cuff 121 in stepwise fashion to different deflation pressures, while pausing at each deflation pressure to capture pressure data over a number of subject 110 pulses. The resulting deflation stage pressure data can be used for blood pressure measurements such as systolic blood pressure, diastolic blood pressure, and MAP.

The "Full Hybrid: Inflate and Deflate" 318 mode can include a mode in which a blood pressure measurement device, such as the blood pressure measurement device 120 illustrated in FIG. 1, is configured to collect cuff pressure data during both the inflate and the deflate stage, without skipping any deflation pressure levels as may be done for the "Fast Hybrid: Inflate and Selective Deflate" 314 mode. Because full data is collected across inflate and deflate, the resulting collected pressure data may have significant overlap and redundancy and as a result may have relatively higher accuracy and reliability than the other modes. The "Full Hybrid: Inflate and Deflate" 318 mode may be particularly useful in noisy environments.

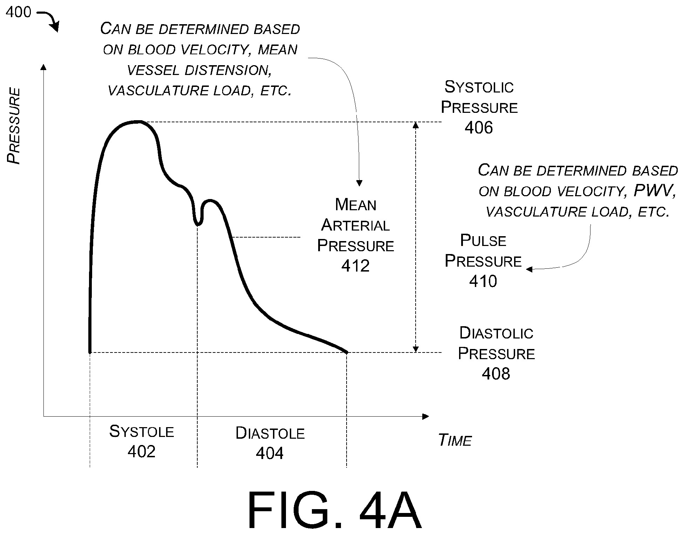

FIG. 4A illustrates an example pressure waveform 400. A horizontal axis represents time, and a vertical axis represents pressure. The waveform 400 of FIG. 4A represents blood pressure over the course of a single cardiac cycle, for instance.

The time axis is divided into two regions representing distinct phases of the cardiac cycle. A first phase represents systole 402 and a second phase represents diastole 404. It should be noted that the systole 402 represented in FIG. 4A may be slightly delayed with respect to the heart being in systole, and the diastole 404 represented in FIG. 4A may be slightly delayed with respect to the heart being in diastole, depending on the distance between the heart and the measurement location. The systole 402 phase of FIG. 4A may represent a change in blood pressure that occurs due to the systole phase of the heart, and the diastole 404 phase of FIG. 4A may represent a change in blood pressure that occurs due to the diastole phase of the heart.

During systole 402, the blood pressure in the blood vessel rises to a peak that represents the systolic pressure 406. Subsequently, the pressure decreases until systole 402 ends. During diastole 404, the blood pressure may rise slightly to a second peak that is lower than the systolic pressure 406. Then, the pressure decays to a diastolic pressure 408. A pulse pressure 410 is defined as the difference between the systolic pressure 406 and the diastolic pressure 408. A MAP 412 is defined as the mean of the waveform 400.

It should be noted that various blood pressure metrics described herein can be derived based on a static component of a blood pressure metric (e.g., a component that does not change over the course of the cardiac cycle) as well as a dynamic component of the blood pressure metric (e.g., a component that changes over the course of the cardiac cycle). MAP and pulse pressure are examples of static and dynamic components, but implementations of the present disclosure are not limited to MAP and pulse pressure.

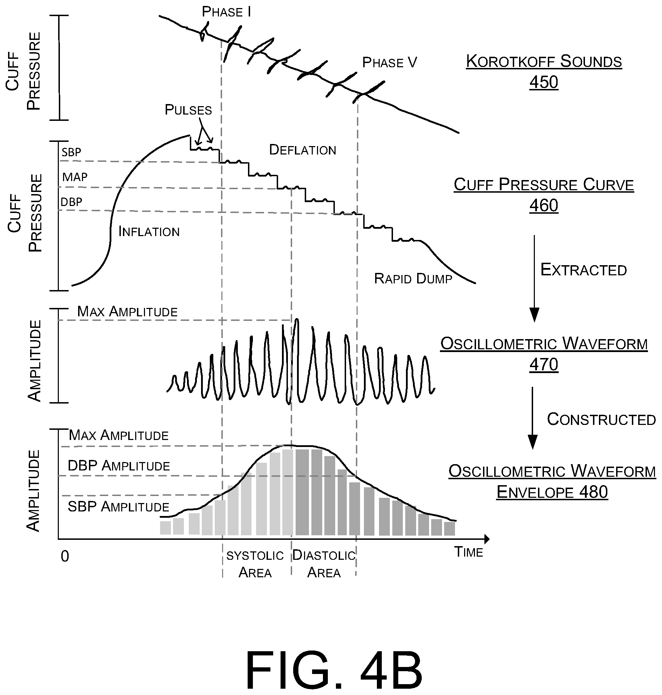

FIG. 4B illustrates an example cuff pressure curve 460, a section of the cuff pressure curve bounded by Korotkoff sounds 450, an oscillometric waveform 470 that can be extracted from the cuff pressure curve 460 and optionally gated according to the techniques described herein, and an oscillometric waveform envelope 480 that can be constructed from the oscillometric waveform 470. While FIG. 4B illustrates the deflation portion of the cuff pressure curve 460 and the corresponding Korotkoff sounds 450, oscillometric waveform 470, and oscillometric waveform envelope 480, and inflation portion of the cuff pressure curve can be used along with another set of Korotkoff sounds to construct an inflation oscillometric waveform and inflation oscillometric waveform envelope.

The cuff pressure curve 460 represents the pressure detected within the inflatable cuff 121 over time during a deflation portion of a blood pressure measurement cycle. As the cuff deflates, the cuff pressure curve 460 decreases from a maximum inflation pressure, as shown. The Korotkoff sounds 450 can be detected during deflation and correspond to turbulent blood flow in the limb of the subject 110 as the cuff pressure passes through the range between systolic blood pressure (SBP) and diastolic blood pressure (DBP). Phase I of the Korotkoff sounds 450 corresponds to the initial appearance of tapping sounds and indicates the SBP. Phase V of the Korotkoff sounds 450 corresponds to the disappearance of sounds and indicates the DBP. The section of the cuff pressure curve 460 bounded by the Korotkoff sounds 450 represents the clinically relevant portion of the deflation cycle for blood pressure determination.

The oscillometric waveform 470 can be extracted from the cuff pressure curve 460 by filtering or otherwise isolating an oscillatory component of the cuff pressure curve. The oscillometric waveform 470 represents pressure oscillations caused by arterial pulses transmitted to the cuff as blood flows through a compressed artery. The amplitude of the oscillometric waveform 470 varies during deflation, with smaller amplitude oscillations at higher cuff pressures near SBP, increasing oscillation amplitudes as cuff pressure approaches mean arterial pressure (MAP), and decreasing oscillation amplitude as cuff pressure decreases toward DBP. The oscillometric waveform 470 can optionally be synthesized with an inflation oscillometric waveform according to the techniques described herein, to remove artifacts and improve the accuracy of blood pressure determinations.

The oscillometric waveform envelope 480 can be constructed from the oscillometric waveform 470 by identifying the peak amplitudes of the oscillations over time. The oscillometric waveform envelope 480 exhibits a characteristic shape with a maximum amplitude corresponding to MAP. The SBP and DBP can be determined from the oscillometric waveform envelope 480 using ratios or other techniques that identify the cuff pressures at which the oscillation amplitude reaches predetermined fractions of the maximum amplitude. By synthesizing the oscillometric waveform 470 with an inflation oscillometric, optionally prior to constructing the oscillometric waveform envelope 480, artifacts can be excluded from the envelope construction, thereby improving the reliability of SBP, DBP, and MAP determinations.

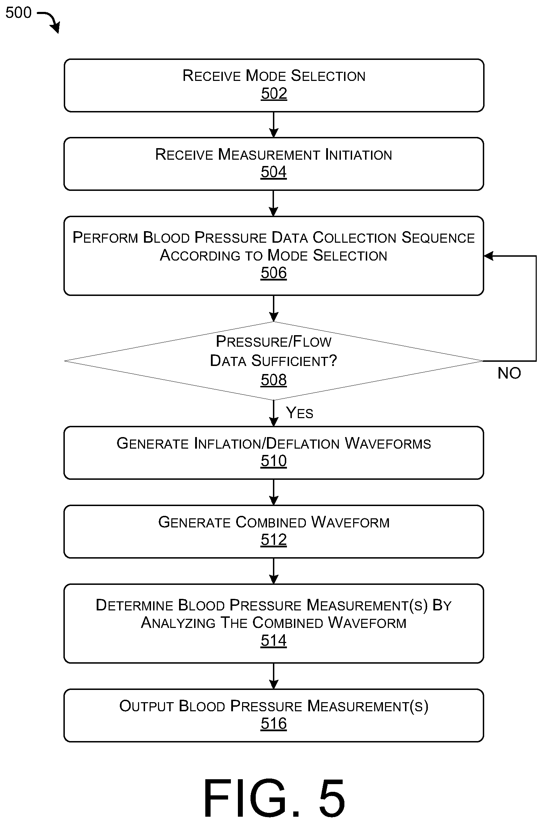

FIG. 5 illustrates an example process 500 for using a combination of inflation and deflation data to noninvasively determine the blood pressure of a subject. In various examples the process 500 is performed by an entity including the blood pressure measurement device 120, the monitor-defibrillator 130, a medical device, a processor, a further (e.g., an additional) computing device, or any combination thereof.

At operation 502, the entity receives a mode selection. For example, the entity may receive a mode selection via a blood pressure measurement mode selection UI 310 such as illustrated in FIG. 3. The blood pressure measurement mode selection UI 310 may be presented via an input device such as a display 300 which is configured to receive an operator input signal, such as a touch activation or other signal, indicating activation of a blood pressure measurement mode, such as any of the example modes illustrated in FIG. 3.

In some implementations, the entity may for example store the mode selection and use the selected mode for all subsequent blood pressure measurements, at least until a different mode selection is made. Alternatively, the entity may treat the mode selection as a measurement initiation as well as a mode selection, and the entity may proceed with a blood pressure measurement according to the selected mode.

At operation 504, the entity may receive a measurement initiation. For example, an operator may press a measurement initiation button or may otherwise indicate a measurement initiation via a display such as the display 132 illustrated in FIG. 1.

At operation 506, the entity may perform a blood pressure data collection sequence according to the mode selection received at operation 502. The entity may inflate an inflatable cuff 121, optionally collecting flow and pressure data during the inflation stage (in accordance with mode selection), and the entity may then deflate the inflatable cuff 121, optionally collecting flow and pressure data during the deflation stage (again in accordance with mode selection).

For example, in the "Fast Hybrid: Inflate and Selective Deflate" 314 mode illustrated in FIG. 3, the entity may inflate the inflatable cuff 121 and collect flow and pressure data during the inflation stage, identify missing portions of the data collected during the inflation stage, and then deflate the inflatable cuff 121 to selected deflation pressures corresponding to the missing portions while collecting deflation stage flow and pressure data. The "Fast Hybrid: Inflate and Selective Deflate" 314 mode may also be referred to herein as a selective deflation mode. The entity may cause the inflatable cuff 121 to deflate to the one or more deflation pressures in response to activation of the selective deflation mode. The entity may determine, by analyzing data indicative of inflation pressure, one or more deflation pressures for use in generating a combined waveform. The entity may cause the inflatable cuff 121 to deflate to the one or more deflation pressures in order to generate data indicating deflation pressure.

Alternatively, in the "Fast: Inflate Only" 312 mode illustrated in FIG. 3, the entity may inflate the inflatable cuff 121 and collect flow and pressure data during the inflation stage, and the entity may then rapidly deflate the inflatable cuff 121 without further data collection. In the "Normal: Deflate Only" 316 mode illustrated in FIG. 3, the entity may inflate the inflatable cuff 121 without collecting flow and pressure data during the inflation stage, and the entity may then deflate the inflatable cuff 121 to all deflation pressures while collecting deflation stage flow and pressure data. In the "Full Hybrid: Inflate and Deflate" 318 mode illustrated in FIG. 3, the entity may inflate the inflatable cuff 121 and collect flow and pressure data during the inflation stage, and the entity may then deflate the inflatable cuff 121 to all deflation pressures while collecting deflation stage flow and pressure data.

At operation 506, the entity may therefore receive data indicating an inflation pressure in an inflatable cuff 121 inflating around a limb of a subject 110 during an inflation time, and the entity may receive data indicating a deflation pressure in the inflatable cuff 121 deflating around the limb of the subject 110 during a deflation time.

At operation 508, the entity may make a determination regarding whether the collected blood pressure measurement data is sufficiently reliable. For example, excess noise which exceeds a noise threshold may result in an unreliable measurement. If the data is sufficient, the process 500 can continue to the subsequent operations. If the data is not sufficient, the process 500 can optionally return to a previous operation such as operation 506, to thereby attempt to repeat data collection.

In an example, operations 506 and 508 may be repeated any number of times, optionally up to a repeat limit, until reliable data is collected. Data collected but determined to be unreliable can be considered additional inflation/deflation data which is discarded in favor of any eventually collected reliable data. The entity can therefore receive additional inflation data indicating additional inflation pressures in the inflatable cuff 121 inflating around the limb of the subject 110 during an additional inflation time or receive additional deflation data indicating additional deflation pressures in the inflatable cuff 121 deflating around the limb of the subject 110 during an additional deflation time. The entity can be configured to discard one or more of the additional inflation data or the additional deflation data in favor or using the eventually collected, reliable portion of the data indicating the inflation pressures and the eventually collected, reliable portion of the data indicating the deflation pressure. The entity can use the reliable data to generate the combined waveform described herein.

At operation 510, the entity may use the data collected at operation 506 to generate inflation and/or deflation waveforms. For example, the entity may use the cuff pressure data 210, illustrated in FIG. 2, to generate the inflation waveform 221 and the deflation waveform 222. In some embodiments, the entity may generate complete waveforms while in other embodiments, the entity may generate partial waveforms.

At operation 512, the entity may generate a combined waveform based on the inflation and deflation waveforms generated at operation 510. The entity may be configured to combine at least a portion of the data indicating the inflation pressure and at least a portion of the data indicating the deflation pressure to generate the combined waveform. This disclosure provides a variety of example techniques that can optionally be applied at operation 512.

In one example, the entity may be configured to generate the combined waveform by reversing the data indicative of the inflation pressure, as reflected in the inflation waveform 221, or the data indicative of the deflation pressure, as reflected in the deflation waveform 222, resulting in alignable waveforms. The entity may then align the alignable waveforms.

In another example, the entity may be configured to generate the combined waveform by combining portions of the inflation waveform 221 and the deflation waveform 222. For instance, portion of data indicative of inflation pressure, namely, a portion of the inflation waveform 221, may correspond to a first pressure range bounded by a particular pressure, and a portion of data indicative of deflation pressure, namely, a portion of the deflation waveform 222, may correspond to a second pressure range bounded by the particular pressure. The entity may be configured to generate the combined waveform at least in part by adjoining the portion of the data indicative of the inflation pressure with the portion of the data indicative of the deflation pressure.

In further examples, the entity may be configured to generate the combined waveform by using any data combination techniques including but not limited to cross-correlating, cross-referencing, cross-checking, superimposing, interpolating or convolving the portion of the data indicative of the inflation pressure with the portion of the data indicative of the deflation pressure. The entity may optionally generate the combined waveform by determining an average of the portion of the data indicative of the inflation pressure and the portion of the data indicative of the deflation pressure.

In another example, the entity may be configured to generate the combined waveform by identifying, in the data indicative of the inflation pressure or the data indicative of the deflation pressure, one or more outlier values, and omitting the one or more outlier values from the portion of the data indicative of the inflation pressure or the portion of the data indicative of the deflation pressure. The resulting "cleaned up" data, without outliers, can be used to generate the combined waveform.

At operation 514, the entity may determine blood pressure measurement(s) by analyzing the combined waveform. For example, the entity may be configured to determine a systolic blood pressure of the subject 110 by analyzing the combined waveform, determine a diastolic blood pressure of the subject 110 by analyzing the combined waveform, and/or determine a MAP of the subject 110 by analyzing the combined waveform.

At operation 516, the entity may cause an output device, e.g., the monitor-defibrillator 130, to output blood pressure measurements determined at operation 514. For example, the entity may output an indication of the systolic blood pressure, an indication of the diastolic blood pressure, and/or an indication of the MAP on the display 132. The indication may include a number value, a graphical / pictorial output, and/or a color output such as a green, yellow, or red color.

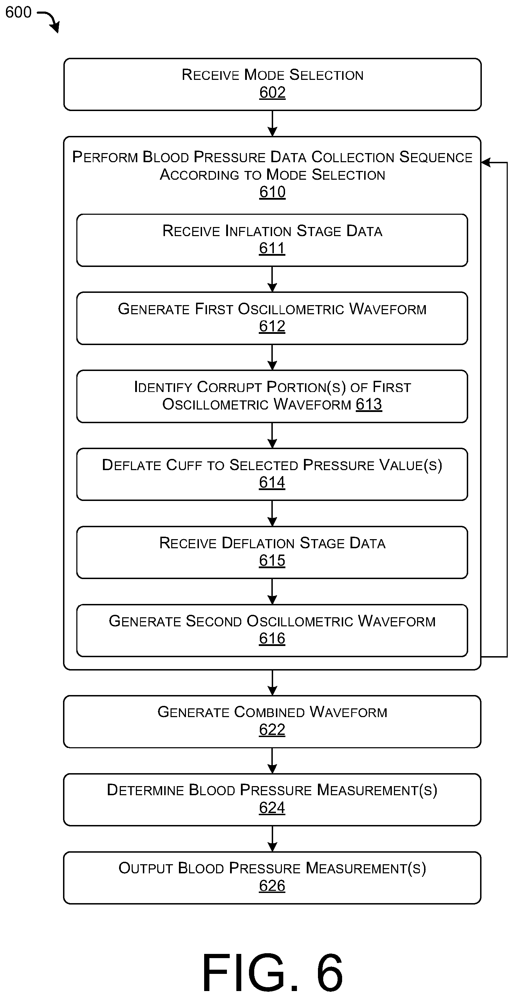

FIG. 6 illustrates another example process 600 for using a combination of inflation and deflation data to noninvasively determine the blood pressure of a subject. The process 600, in numerous examples, is performed by an entity including the blood pressure measurement device 120, the monitor-defibrillator 130, a medical device, a processor, a further (e.g., an additional) computing device, or any combination thereof. In some implementations, a medical device such as the monitor-defibrillator 130 may include at least one electrocardiogram (ECG) monitoring circuit and at least one defibrillator circuit, in addition to the blood pressure measurement features described herein.

At operation 602, the entity may receive a mode selection, e.g., via a display 300 such as illustrated in FIG. 3. While any mode may be selected, FIG. 6 will describe an example in which the "Fast Hybrid: Inflate and Selective Deflate" 314 mode is selected. This mode may also be referred to herein as a selective deflation mode. At operation 602, an operator input may activate the selective deflation mode wherein a blood pressure cuff is selectively deflated to selected pressure values. Operator inputs received via the display 300 can also optionally deactivate the selective deflation mode, as will be appreciated.

At operation 610, the entity may perform a blood pressure data collection sequence according to the mode selection received at operation 602. Operation 610 can include operations 611, 612, 613, 614, 615, and 616. If the blood pressure data collection sequence does not result in usable data, then the operation 610 can be repeated as many times as needed, resulting in receiving one or more additional blood pressure signals, evaluating the one or more additional blood pressure signals, and discarding the one or more additional blood pressure signals in favor or using, e.g., the first blood pressure signals and the second blood pressure signals described in connection with operations 611, 612, 613, 614, 615, and 616 to produce a combined oscillometric waveform.

At operation 611, the entity may receive inflation stage data, e.g., first blood pressure signals from a cuff inflation stage of a blood pressure cuff. At operation 612, the first blood pressure signals can be used to generate or form a first oscillometric waveform.

At operation 613, in accordance with the selective deflation mode selected at operation 602, the entity may identify a corrupt portion of the first oscillometric waveform. At least one first segment of the first oscillometric waveform, which can eventually be used to generate a combined waveform, need not include the corrupt portion of the first oscillometric waveform.

At operation 614, the entity can control deflation of the blood pressure cuff by selectively deflating the blood pressure cuff to at least one selected pressure value which is selected based on the corrupt portion of the first oscillometric waveform, namely, the portion identified at operation 613. At operation 615, the entity can receive deflation stage data, e.g., second blood pressure signals from a cuff deflation stage of the blood pressure cuff.

At operation 616, the second blood pressure signals can be used to generate or form a second oscillometric waveform. In accordance with the selective deflation mode selected at operation 602, the entity may eventually use at least one second segment of the second oscillometric waveform to replace the corrupt portion of the first oscillometric waveform in the combined oscillometric waveform.

At operation 622, the entity can generate a combined waveform by combining the first oscillometric waveform and the oscillometric waveform, or segments/portions thereof, to generate the combined waveform. In some examples, the entity can combine at least one first segment of the first oscillometric waveform (e.g., the uncorrupted portion of the first oscillometric waveform) with at least one second segment of the second oscillometric waveform (e.g., the portions generated at the selected deflation pressures) to produce the combined oscillometric waveform.

In some implementations, generating the combined waveform at operation 622 can include determining one or more combined oscillometric waveform values. The combined oscillometric waveform values can be based on one or more combinations of first oscillometric waveform values, associated with the first oscillometric waveform, and second oscillometric waveform values, associated with the second oscillometric waveform. The combined oscillometric waveform can include the one or more combined oscillometric waveform values.

In another example, generating the combined waveform at operation 622 can include cross-correlating, cross-referencing, cross-checking, superimposing, interpolating, or convolving the first oscillometric waveform or the second oscillometric waveform in order to combine at least one first segment of the first oscillometric waveform with at least one second segment of the second oscillometric waveform. Any other combination techniques can be applied, and this disclosure is not limited to any particular technique.

At operation 624, the entity can determine blood pressure measurement(s) based on the combined oscillometric waveform. For example, MAP, systolic, and/or diastolic blood pressure measurements can be determined. At operation 626, the entity can output a graphical element representing the blood pressure measurement, such as a text display, pictorial display, color display, audio display or other output.

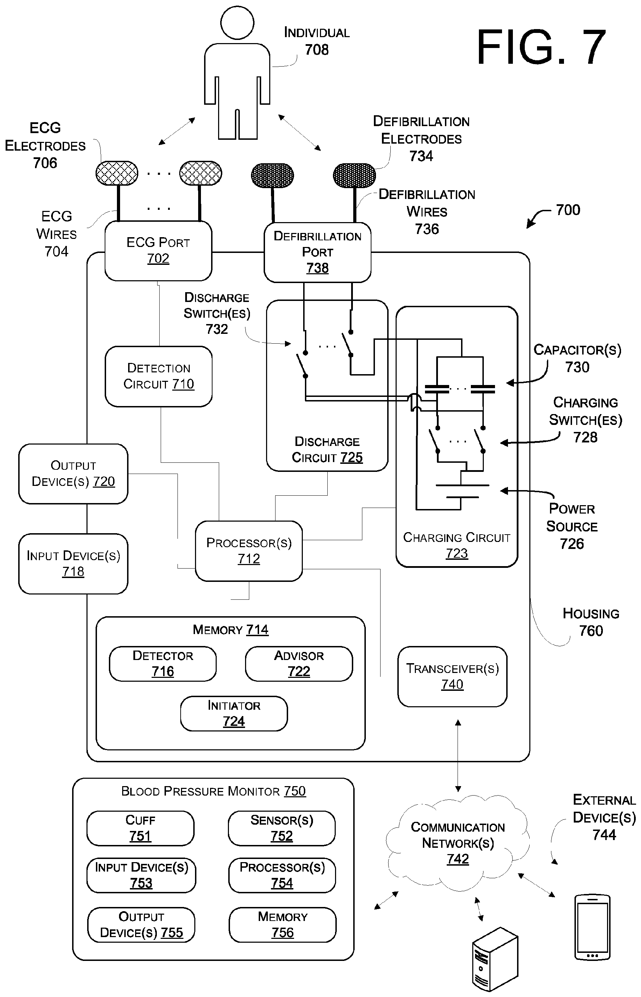

FIG. 7 illustrates an example of an external defibrillator 700 configured to perform various functions described herein. For example, the external defibrillator 700 can implement the monitor-defibrillator 130 described above with reference to FIG. 1.

The external defibrillator 700 includes an ECG port 702 connected to multiple ECG wires 704. In some cases, the ECG wires 704 are removeable from the ECG port 702. For instance, the ECG wires 704 are plugged into the ECG port 702 via connectors. The ECG wires 704 are connected to ECG electrodes 706, respectively. In various implementations, the ECG electrodes 706 are disposed on distinct locations on an individual 708. A detection circuit 710 is configured to detect relative voltages between the ECG electrodes 706. These voltages are indicative of the electrical activity of the heart of the individual 708.

In various implementations, the ECG electrodes 706 are in contact with the distinct locations on the skin of the individual 708. In some examples, a first one of the ECG electrodes 706 is placed on the skin between the heart and right arm of the individual 708, a second one of the ECG electrodes 706 is placed on the skin between the heart and left arm of the individual 708, and a third one of the ECG electrodes 706 is placed on the skin between the heart and a leg (either the left leg or the right leg) of the individual 708. In these examples, the detection circuit 710 is configured to measure the relative voltages between the first, second, and third ECG electrodes 706. Respective pairings of the ECG electrodes 706 are referred to as "leads," and the voltages between the pairs of ECG electrodes 706 are known as "lead voltages." In some examples, more than three ECG electrodes 706 are included, such that 5-lead or 12-lead ECG signals are detected by the detection circuit 710.

The detection circuit 710 includes at least one analog circuit, at least one digital circuit, or a combination thereof. The detection circuit 710 receives the analog electrical signals from the ECG electrodes 706, via the ECG port 702 and the ECG wires 704. In some cases, the detection circuit 710 includes one or more analog filters configured to filter noise and/or artifact from the electrical signals. The detection circuit 710 includes an analog-to-digital (ADC) in various examples. The detection circuit 710 generates a digital signal indicative of the analog electrical signals from the ECG electrodes 706. This digital signal can be referred to as an "ECG signal" or an "ECG."

In some cases, the detection circuit 710 further detects an electrical impedance between at least one pair of the ECG electrodes 706. For example, the detection circuit 710 includes, or otherwise controls, a power source that applies a known voltage (or current) across a pair of the ECG electrodes 706 and detects a resultant current (or voltage) between the pair of the ECG electrodes 706. The impedance is generated based on the applied signal (voltage or current) and the resultant signal (current or voltage). In various cases, the impedance corresponds to respiration of the individual 708, chest compressions performed on the individual 708, and other physiological states of the individual 708. In various examples, the detection circuit 710 includes one or more analog filters configured to filter noise and/or artifact from the resultant signal. The detection circuit 710 generates a digital signal indicative of the impedance using an ADC. This digital signal can be referred to as an "impedance signal" or an "impedance."

The detection circuit 710 provides the ECG signal and/or the impedance signal to one or more processors 712 in the external defibrillator 700. In some implementations, the processor(s) 712 includes a central processing unit (CPU), a graphics processing unit (GPU), both CPU and GPU, or other processing unit or component known in the art.

The processor(s) 712 is operably connected to memory 714. In various implementations, the memory 714 is volatile (such as random access memory (RAM)), nonvolatile (such as read only memory (ROM), flash memory, etc.) or some combination of the two. The memory 714 stores instructions that, when executed by the processor(s) 712, causes the processor(s) 712 to perform various operations. In various examples, the memory 714 stores methods, threads, processes, applications, objects, modules, any other sort of executable instruction, or a combination thereof. In some cases, the memory 714 stores files, databases, or a combination thereof. In some examples, the memory 714 includes, but is not limited to, RAM, ROM, electrically erasable programmable read-only memory (EEPROM), flash memory, or any other memory technology. In some examples, the memory 714 includes one or more of CD-ROMs, digital versatile discs (DVDs), content-addressable memory (CAM), or other optical storage, magnetic cassettes, magnetic tape, magnetic disk storage or other magnetic storage devices, or any other medium which can be used to store the desired information and which can be accessed by the processor(s) 712 and/or the external defibrillator 700. In some cases, the memory 714 at least temporarily stores the ECG signal and/or the impedance signal.

In various examples, the memory 714 includes a detector 716, which causes the processor(s) 712 to determine, based on the ECG signal and/or the impedance signal, whether the individual 708 is exhibiting a particular heart rhythm. For instance, the processor(s) 712 determines whether the individual 708 is experiencing a shockable rhythm that is treatable by defibrillation. Examples of shockable rhythms include ventricular fibrillation (VF) and pulseless ventricular tachycardia (VT). In some examples, the processor(s) 712 determines whether any of a variety of different rhythms (e.g., asystole, sinus rhythm, atrial fibrillation (AF), etc.) are present in the ECG signal.

The processor(s) 712 is operably connected to one or more input devices 718 and one or more output devices 720. Collectively, the input device(s) 718 and the output device(s) 720 function as an interface between a user and the defibrillator 700. The input device(s) 718 is configured to receive an input from a user and includes at least one of a keypad, a cursor control, a touch-sensitive display, a voice input device (e.g., a microphone), a haptic feedback device (e.g., a gyroscope), or any combination thereof. The output device(s) 720 includes at least one of a display, a speaker, a haptic output device, a printer, or any combination thereof. In various examples, the processor(s) 712 causes a display among the input device(s) 718 to visually output a waveform of the ECG signal and/or the impedance signal. In some implementations, the input device(s) 718 includes one or more touch sensors, the output device(s) 720 includes a display screen, and the touch sensor(s) are integrated with the display screen. Thus, in some cases, the external defibrillator 700 includes a touchscreen configured to receive user input signal(s) and visually output physiological parameters, such as the ECG signal and/or the impedance signal.

In some examples, the memory 714 includes an advisor 722, which, when executed by the processor(s) 712, causes the processor(s) 712 to generate advice and/or control the output device(s) 720 to output the advice to a user (e.g., a rescuer). In some examples, the processor(s) 712 provides, or causes the output device(s) 720 to provide, an instruction to perform CPR on the individual 708. In some cases, the processor(s) 712 evaluates, based on the ECG signal, the impedance signal, or other physiological parameters (e.g., one or more blood pressure parameters), CPR being performed on the individual 708 and causes the output device(s) 720 to provide feedback about the CPR in the instruction. According to some examples, the processor(s) 712, upon identifying that a shockable rhythm is present in the ECG signal, causes the output device(s) 720 to output an instruction and/or recommendation to administer a defibrillation shock to the individual 708.

The memory 714 also includes an initiator 724 which, when executed by the processor(s) 712, causes the processor(s) 712 to control other elements of the external defibrillator 700 in order to administer a defibrillation shock to the individual 708. In some examples, the processor(s) 712 executing the initiator 724 selectively causes the administration of the defibrillation shock based on determining that the individual 708 is exhibiting the shockable rhythm and/or based on an input from a user (received, e.g., by the input device(s) 718. In some cases, the processor(s) 712 causes the defibrillation shock to be output at a particular time, which is determined by the processor(s) 712 based on the ECG signal and/or the impedance signal.

The processor(s) 712 is operably connected to a charging circuit 723 and a discharge circuit 725. In various implementations, the charging circuit 723 includes a power source 726, one or more charging switches 728, and one or more capacitors 730. The power source 726 includes, for instance, a battery. The processor(s) 712 initiates a defibrillation shock by causing the power source 726 to charge at least one capacitor among the capacitor(s) 730. For example, the processor(s) 712 activates at least one of the charging switch(es) 728 in the charging circuit 723 to complete a first circuit connecting the power source 726 and the capacitor to be charged. Then, the processor(s) 712 causes the discharge circuit 725 to discharge energy stored in the charged capacitor across a pair of defibrillation electrodes 734, which are in contact with the individual 708. For example, the processor(s) 712 deactivates the charging switch(es) 728 completing the first circuit between the capacitor(s) 730 and the power source 726 and activates one or more discharge switches 732 completing a second circuit connecting the charged capacitor 730 and at least a portion of the individual 708 disposed between defibrillation electrodes 734.

The energy is discharged from the defibrillation electrodes 734 in the form of a defibrillation shock. For example, the defibrillation electrodes 734 are connected to the skin of the individual 708 and located at positions on different sides of the heart of the individual 708, such that the defibrillation shock is applied across the heart of the individual 708. The defibrillation shock, in various examples, depolarizes a significant number of heart cells in a short amount of time. The defibrillation shock, for example, interrupts the propagation of the shockable rhythm (e.g., VF or VT) through the heart. In some examples, the defibrillation shock is 200 J or greater with a duration of about 0.015 seconds. In some cases, the defibrillation shock has a multiphasic (e.g., biphasic) waveform. The discharge switch(es) 732 are controlled by the processor(s) 712, for example. In various implementations, the defibrillation electrodes 734 are connected to defibrillation wires 736. The defibrillation wires 736 are connected to a defibrillation port 738, in implementations. According to various examples, the defibrillation wires 736 are removable from the defibrillation port 738. For example, the defibrillation wires 736 are plugged into the defibrillation port 738.

In various implementations, the processor(s) 712 is operably connected to one or more transceivers 740 that transmit and/or receive data over one or more communication networks 742. For example, the transceiver(s) 740 includes a network interface card (NIC), a network adapter, a local area network (LAN) adapter, or a physical, virtual, or logical address to connect to the various external devices and/or systems. In various examples, the transceiver(s) 740 includes any sort of wireless transceivers capable of engaging in wireless communication (e.g., radio frequency (RF) communication). For example, the communication network(s) 742 includes one or more wireless networks that include a 3^{rd} Generation Partnership Project (3GPP) network, such as a Long Term Evolution (LTE) radio access network (RAN) (e.g., over one or more LTE bands), a New Radio (NR) RAN (e.g., over one or more NR bands), or a combination thereof. In some cases, the transceiver(s) 740 includes other wireless modems, such as a modem for engaging in WI-FI^{®}, WIGIG^{®}, WIMAX^{®}, BLUETOOTH^{®}, or infrared communication over the communication network(s) 742.

The defibrillator 700 is configured to transmit and/or receive data (e.g., ECG data, blood pressure data, impedance data, data indicative of one or more detected heart rhythms of the individual 708, data indicative of one or more defibrillation shocks administered to the individual 708, etc.) with one or more external devices 744 via the communication network(s) 742. The external devices 744 include, for instance, mobile devices (e.g., mobile phones, smart watches, etc.), Internet of Things (IoT) devices, medical devices, computers (e.g., laptop devices, servers, etc.), or any other type of computing device configured to communicate over the communication network(s) 742. In some examples, the external device(s) 744 is located remotely from the defibrillator 700, such as at a remote clinical environment (e.g., a hospital). According to various implementations, the processor(s) 712 causes the transceiver(s) 740 to transmit data to the external device(s) 744. In some cases, the transceiver(s) 740 receives data from the external device(s) 744 and the transceiver(s) 740 provide the received data to the processor(s) 712 for further analysis.

According to various cases, the external device(s) 744 can include a blood pressure monitor 750 configured to non-invasively detect a blood pressure of the individual 708. In some examples, the blood pressure monitor 750 is the blood pressure measurement device 120 described above with reference to FIG. 1. The blood pressure monitor 750 includes, for example, a cuff 751, sensor(s) 752, input device(s) 753, processor(s) 754, output device(s) 755, and memory 756. The illustrated components of the blood pressure monitor 750 can implement the components of the blood pressure measurement device 120. For example, the cuff 751 can implement the inflatable cuff 121, the pump 122, and the vent 123. The sensor(s) 752 can implement the pressure sensor 124 and the flow sensor 125. The processor(s) 754 and memory 756 can implement the controller 126 and the inflate/deflate waveform combiner 127.

The blood pressure monitor 750, in various cases, includes at least one processor 754 configured to execute instructions stored in memory 756, thereby performing various functions described herein. In some examples, the processor(s) 754 is configured to inflate and deflate the cuff 751, gather pressure data via sensor(s) 752 which may be bounded by or otherwise correlated with flow data gathered via sensor(s) 752, generate inflate/deflate oscillometric waveforms, combine the oscillometric waveforms, and use the resulting combined oscillometric waveform to make blood pressure measurement determinations. Any type of processor and/or memory described elsewhere herein can be suitable as the processor(s) 754 and/or memory 756 of the blood pressure monitor 750.

In various cases, the blood pressure monitor 750 further includes one or more transceivers to transmit and/or receive communication signals with the transceiver(s) 740 of the defibrillator 700 over the communication network(s) 742. In some examples, the blood pressure monitor 750 further includes at least one input device 753 (e.g., a button, a touchscreen, a microphone, etc.) and at least one output device 755 (e.g., a display, a speaker, etc.). Any type of input device and/or output device described herein can be included in the input device(s) 753 and/or output device(s) 755. In some examples, the input device(s) 753 includes an accelerometer and/or an additional blood pressure sensor (e.g., an additional blood pressure cuff, pressure gauge, oscillometric blood pressure sensor, or any combination thereof).

In particular examples, the blood pressure monitor 750 transmits an indication of a blood pressure of the individual 708 to the defibrillator 700 via a communication signal. In some cases, the defibrillator 700 performs various functions based on the blood pressure reported by the blood pressure monitor 750. For example, the detector 716 may include instructions to detect pulseless VT in response to detecting VT in the ECG of the individual 708 as well as in response to determining that the blood pressure detected by the blood pressure monitor 750 is below a threshold blood pressure. In various cases, the advisor 722 includes instructions that cause the processor(s) 712 to output a recommendation to administer an electrical shock to the individual 708 in response to detecting the pulseless VT. In some cases, the output device(s) 720 of the defibrillator 700 output an indication of the blood pressure detected by the blood pressure monitor 750.

In various implementations, the external defibrillator 700 also includes a housing 760 that at least partially encloses other elements of the external defibrillator 700. For example, the housing 760 encloses the detection circuit 710, the processor(s) 712, the memory 714, the charging circuit 723, the transceiver(s) 740, or any combination thereof. In some cases, the input device(s) 718 and output device(s) 720 extend from an interior space at least partially surrounded by the housing 760 through a wall of the housing 760. In various examples, the housing 760 acts as a barrier to moisture, electrical interference, and/or dust, thereby protecting various components in the external defibrillator 700 from damage.

In some implementations, the external defibrillator 700 is an automated external defibrillator (AED) operated by an untrained user (e.g., a bystander, layperson, etc.) and can be operated in an automatic mode. In automatic mode, the processor(s) 712 automatically identifies a rhythm in the ECG signal, makes a decision whether to administer a defibrillation shock, charges the capacitor(s) 730, discharges the capacitor(s) 730, or any combination thereof. In some cases, the processor(s) 712 controls the output device(s) 720 to output (e.g., display) a simplified user interface to the untrained user. For example, the processor(s) 712 refrains from causing the output device(s) 720 to display a waveform of the ECG signal and/or the impedance signal to the untrained user, in order to simplify operation of the external defibrillator 700.

In some examples, the external defibrillator 700 is a monitor-defibrillator utilized by a trained user (e.g., a clinician, an emergency responder, etc.) and can be operated in a manual mode or the automatic mode. When the external defibrillator 700 operates in manual mode, the processor(s) 712 cause the output device(s) 720 to display a variety of information that may be relevant to the trained user, such as waveforms indicating the ECG data and/or impedance data, notifications about detected heart rhythms, and the like.

### EXAMPLE CLAUSES

The following Clauses provide various examples of the present disclosure:
1. A medical device, including: a cuff adapted to be disposed around a limb of a subject; a pump configured to inflate the cuff during an inflation time, the subject exhibiting first pulses during the inflation time; a vent configured to deflate the cuff during a deflation time, the subject exhibiting second pulses during the deflation time; a sensor configured to: detect an inflation pressure in the cuff over time during the inflation time; and detect a deflation pressure in the cuff over time during the deflation time; a display; and a processor configured to: cause the pump to inflate the cuff during the inflation time; cause the vent to deflate the cuff during the deflation time; generate a combined waveform by combining at least a portion of data indicative of the inflation pressure in the cuff over time and at least a portion of data indicative of the deflation pressure in the cuff over time; determine a blood pressure of the subject by analyzing the combined waveform; and cause the display to visually present an indication of the blood pressure.
2. The medical device of clause 1, wherein the processor is configured to generate the combined waveform by: reversing the data indicative of the inflation pressure or the data indicative of the deflation pressure, resulting in alignable waveforms; and aligning the alignable waveforms.
3. The medical device of any of clauses 1 to 2, wherein the processor is configured to generate the combined waveform by: identifying, in the data indicative of the inflation pressure or the data indicative of the deflation pressure, one or more outlier values, wherein the at least a portion of the data indicative of the inflation pressure or the at least a portion of the data indicative of the deflation pressure omits the one or more outlier values.
4. A medical device, including: an output device; and a processor configured to: receive data indicating an inflation pressure in a cuff inflating around a limb of a subject during an inflation time; receive data indicating a deflation pressure in the cuff deflating around the limb of the subject during a deflation time; generate a combined waveform by combining at least a portion of the data indicating the inflation pressure and at least a portion of the data indicating the deflation pressure; determine a systolic blood pressure of the subject by analyzing the combined waveform; determine a diastolic blood pressure of the subject by analyzing the combined waveform; determine a mean arterial pressure of the subject by analyzing the combined waveform; cause the output device to output an indication of the systolic blood pressure; and cause the output device to output an indication of the diastolic blood pressure; and cause the output device to output an indication of the mean arterial pressure.
5. The medical device of clause 4, wherein the processor is configured to generate the combined waveform by: reversing the data indicative of the inflation pressure or the data indicative of the deflation pressure, resulting in alignable waveforms; and aligning the alignable waveforms.
6. The medical device of any of clauses 4 to 5, wherein: the portion of the data indicative of the inflation pressure corresponds to a first pressure range bounded by a particular pressure, the portion of the data indicative of the deflation pressure corresponds to a second pressure range bounded by the particular pressure, and wherein the processor is configured to generate the combined waveform at least in part by adjoining the portion of the data indicative of the inflation pressure with the portion of the data indicative of the deflation pressure.
7. The medical device of any of clauses 4 to 6, wherein the processor is configured to generate the combined waveform by cross-correlating, cross-referencing, cross-checking, superimposing, interpolating, or convolving the portion of the data indicative of the inflation pressure with the portion of the data indicative of the deflation pressure.
8. The medical device of any of clauses 4 to 7, wherein the processor is configured to generate the combined waveform by determining an average of the portion of the data indicative of the inflation pressure and the portion of the data indicative of the deflation pressure.
9. The medical device of any of clauses 4 to 8, wherein the processor is configured to generate the combined waveform by: identifying, in the data indicative of the inflation pressure or the data indicative of the deflation pressure, one or more outlier values; and omitting the one or more outlier values from the portion of the data indicative of the inflation pressure or the portion of the data indicative of the deflation pressure.
10. The medical device of any of clauses 4 to 9, wherein the processor is further configured to: determine, by analyzing the data indicative of the inflation pressure, one or more deflation pressures for use in generating the combined waveform; and cause the cuff to deflate to the one or more deflation pressures in order to generate the data indicating the deflation pressure.
11. The medical device of clause 10, further including: an input device configured to receive an operator input signal indicating activation of a selective deflation mode, wherein the processor is configured to cause the cuff to deflate to the one or more deflation pressures in response to the activation of the selective deflation mode.
12. The medical device of any of clauses 4 to 11, wherein the processor further configured to: receive additional inflation data indicating additional inflation pressures in the cuff inflating around the limb of the subject during an additional inflation time; or receive additional deflation data indicating additional deflation pressures in the cuff deflating around the limb of the subject during an additional deflation time; and discarding one or more of the additional inflation data or the additional deflation data in favor or using the portion of the data indicating the inflation pressure and the portion of the data indicating the deflation pressure to generate the combined waveform.
13. The medical device of any of clauses 4 to 12, wherein the processor is further configured to use one or more features of the data indicating the inflation pressure to filter or gate the data indicating the deflation pressure.
14. A method, including: receiving data indicating an inflation pressure in a cuff inflating around a limb of a subject during an inflation time; receiving data indicating a deflation pressure in the cuff deflating around the limb of the subject during a deflation time; generating a combined waveform by combining at least a portion of the data indicating the inflation pressure and at least a portion of the data indicating the deflation pressure; determining a systolic blood pressure of the subject by analyzing the combined waveform; determining a diastolic blood pressure of the subject by analyzing the combined waveform; determining a mean arterial pressure of the subject by analyzing the combined waveform; causing the output device to output an indication of the systolic blood pressure; causing the output device to output an indication of the diastolic blood pressure; and causing the output device to output an indication of the mean arterial pressure.
15. The method of clause 14, further including: reversing the data indicative of the inflation pressure or the data indicative of the deflation pressure, resulting in alignable waveforms; and aligning the alignable waveforms.
16. The method of any of clauses 14 to 15, wherein: the portion of the data indicative of the inflation pressure corresponds to a first pressure range bounded by a particular pressure, the portion of the data indicative of the deflation pressure corresponds to a second pressure range bounded by the particular pressure, and generating the combined waveform includes adjoining the portion of the data indicative of the inflation pressure with the portion of the data indicative of the deflation pressure.
17. The method of any of clauses 14 to 16, wherein generating the combined waveform includes cross-correlating, cross-referencing, cross-checking, superimposing, interpolating, or convolving the portion of the data indicative of the inflation pressure with the portion of the data indicative of the deflation pressure.
18. The method of any of clauses 14 to 17, wherein generating the combined waveform includes determining an average of the portion of the data indicative of the inflation pressure and the portion of the data indicative of the deflation pressure.
19. The method of any of clauses 14 to 18, wherein generating the combined waveform includes: identifying, in the data indicative of the inflation pressure or the data indicative of the deflation pressure, one or more outlier values; and omitting the one or more outlier values from the portion of the data indicative of the inflation pressure or the portion of the data indicative of the deflation pressure.
20. The method of any of clauses 14 to 19, further including: determining, by analyzing the data indicative of the inflation pressure, one or more deflation pressures for use in generating the combined waveform; and causing the cuff to deflate to the one or more deflation pressures in order to generate the data indicating the deflation pressure.
21. The method of clause 20, further including receiving an operator input signal indicating activation of a selective deflation mode, wherein causing the cuff to deflate to the one or more deflation pressures is in response to the activation of the selective deflation mode.
22. The method of any of clauses 14 to 21, wherein generating the combined waveform comprises using one or more features of the data indicating the inflation pressure to filter or gate the data indicating the deflation pressure.
23. A medical device, including: at least one blood pressure input adapted to receive blood pressure signals from at least one inflatable and deflatable blood pressure cuff deployed on a subject; a display; and one or more processors configured to: determine at least one blood pressure measurement based on the blood pressure signals; and cause the display to visually present the at least at least one blood pressure measurement; wherein determining the at least one blood pressure measurement based on the blood pressure signals includes: receiving first blood pressure signals during a cuff inflation stage of the blood pressure cuff, wherein the first blood pressure signals form a first oscillometric waveform; receiving second blood pressure signals during a cuff deflation stage of the blood pressure cuff, wherein the second blood pressure signals form a second oscillometric waveform; combining at least one first segment of the first oscillometric waveform with at least one second segment of the second oscillometric waveform to produce a combined oscillometric waveform; and determining the at least one blood pressure measurement based on the combined oscillometric waveform.
24. The medical device of clause 23, further including identifying a corrupt portion of the first oscillometric waveform, wherein the at least one first segment of the first oscillometric waveform does not include the corrupt portion of the first oscillometric waveform, and wherein the at least one second segment of the second oscillometric waveform replaces the corrupt portion of the first oscillometric waveform in the combined oscillometric waveform.
25. The medical device of any of clauses 23 to 24, further including controlling deflation of the blood pressure cuff by selectively deflating the blood pressure cuff to at least one selected pressure value which is selected based on a corrupt portion of the first oscillometric waveform.
26. A medical device, including: one or more processors configured to determine a blood pressure measurement, wherein determining the blood pressure measurement includes:
   receiving first blood pressure signals from a cuff inflation stage of a blood pressure cuff,
   wherein the first blood pressure signals form a first oscillometric waveform; receiving second blood pressure signals from a cuff deflation stage of the blood pressure cuff, wherein the second blood pressure signals form a second oscillometric waveform; combining at least one first segment of the first oscillometric waveform with at least one second segment of the second oscillometric waveform to produce a combined oscillometric waveform; and
   determining the at least one blood pressure measurement based on the combined oscillometric waveform.
27. The medical device of clause 26, further including identifying a corrupt portion of the first oscillometric waveform, wherein the at least one first segment of the first oscillometric waveform does not include the corrupt portion of the first oscillometric waveform, and wherein the at least one second segment of the second oscillometric waveform replaces the corrupt portion of the first oscillometric waveform in the combined oscillometric waveform.
28. The medical device of clause 27, further including controlling deflation of the blood pressure cuff by selectively deflating the blood pressure cuff to at least one selected pressure value which is selected based on the corrupt portion of the first oscillometric waveform.
29. The medical device of clause 28, wherein determining the blood pressure measurement further includes receiving an operator input to activate and deactivate a selective deflation mode wherein the blood pressure cuff is selectively deflated to the selected pressure values.
30. The medical device of any of clauses 26 to 29, wherein determining the blood pressure measurement further includes receiving one or more additional blood pressure signals, evaluating the one or more additional blood pressure signals, and discarding the one or more additional blood pressure signals in favor or using the first blood pressure signals and the second blood pressure signals to produce the combined oscillometric waveform.
31. The medical device of any of clauses 26 to 30, wherein determining the blood pressure measurement further includes cross-correlating, cross-referencing, cross-checking, superimposing, interpolating or convolving the first oscillometric waveform or the second oscillometric waveform in order to combine the at least one first segment of the first oscillometric waveform with the at least one second segment of the second oscillometric waveform.
32. The medical device of any of clauses 26 to 31, further including determining one or more combined oscillometric waveform values based on one or more combinations of first oscillometric waveform values associated with the first oscillometric waveform with second oscillometric waveform values associated with the second oscillometric waveform, and wherein the combined oscillometric waveform includes the one or more combined oscillometric waveform values.
33. The medical device of any of clauses 26 to 32, wherein the medical device further includes at least one electrocardiogram (ECG) monitoring circuit and at least one defibrillator circuit.
34. The medical device of any of clauses 26 to 33, wherein the medical device is further configured to output a graphical element representing the blood pressure measurement.
35. A method, including: receiving first blood pressure signals from a cuff inflation stage of a blood pressure cuff, wherein the first blood pressure signals form a first oscillometric waveform; receiving second blood pressure signals from a cuff deflation stage of the blood pressure cuff, wherein the second blood pressure signals form a second oscillometric waveform; combining at least one first segment of the first oscillometric waveform with at least one second segment of the second oscillometric waveform to produce a combined oscillometric waveform; and determining at least one blood pressure measurement based on the combined oscillometric waveform.
36. The method of clause 35, further including identifying a corrupt portion of the first oscillometric waveform, wherein the at least one first segment of the first oscillometric waveform does not include the corrupt portion of the first oscillometric waveform, and wherein the at least one second segment of the second oscillometric waveform replaces the corrupt portion of the first oscillometric waveform in the combined oscillometric waveform.
37. The method of clause 36, further including controlling deflation of the blood pressure cuff by selectively deflating the blood pressure cuff to at least one selected pressure value which is selected based on the corrupt portion of the first oscillometric waveform.
38. The method of clause 37, wherein determining the blood pressure measurement further includes receiving an operator input to activate and deactivate a selective deflation mode wherein the blood pressure cuff is selectively deflated to the selected pressure values.
39. The method of any of clauses 35 to 38, wherein determining the blood pressure measurement further includes receiving one or more additional blood pressure signals, evaluating the one or more additional blood pressure signals, and discarding the one or more additional blood pressure signals in favor of using the first blood pressure signals and the second blood pressure signals to produce the combined oscillometric waveform.
40. The method of any of clauses 35 to 39, wherein determining the blood pressure measurement further includes cross-correlating, cross-referencing, cross-checking, superimposing, interpolating or convolving the first oscillometric waveform or the second oscillometric waveform in order to combine the at least one first segment of the first oscillometric waveform with the at least one second segment of the second oscillometric waveform.
41. The method of any of clauses 35 to 40, further including determining one or more combined oscillometric waveform values based on one or more combinations of first oscillometric waveform values associated with the first oscillometric waveform with second oscillometric waveform values associated with the second oscillometric waveform, and wherein the combined oscillometric waveform includes the one or more combined oscillometric waveform values.
42. The method of any of clauses 35 to 41, further including outputting a graphical element representing the blood pressure measurement.
43. A medical device, comprising: a cuff adapted to be disposed around a limb of a subject; a pump configured to inflate the cuff during an inflation time, the subject exhibiting first pulses during the inflation time; a vent configured to deflate the cuff during a deflation time, the subject exhibiting second pulses during the deflation time; a sensor configured to:
   detect an inflation pressure in the cuff over time during the inflation time; and detect a deflation pressure in the cuff over time during the deflation time; a display; and a processor configured to: cause the pump to inflate the cuff during the inflation time; cause the vent to deflate the cuff during the deflation time; generate a combined waveform by combining at least a portion of data indicative of the inflation pressure in the cuff over time and at least a portion of data indicative of the deflation pressure in the cuff over time; determine a blood pressure of the subject by analyzing the combined waveform; and cause the display to visually present an indication of the blood pressure.
44. The medical device of clause 43, wherein the processor is configured to generate the combined waveform by: reversing the data indicative of the inflation pressure or the data indicative of the deflation pressure, resulting in alignable waveforms; and aligning the alignable waveforms.
45. The medical device of clause 43, wherein the processor is configured to generate the combined waveform by: identifying, in the data indicative of the inflation pressure or the data indicative of the deflation pressure, one or more outlier values, wherein the at least a portion of the data indicative of the inflation pressure or the at least a portion of the data indicative of the deflation pressure omits the one or more outlier values.

### CONCLUSION

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be used for realizing implementations of the disclosure in diverse forms thereof.

As will be understood by one of ordinary skill in the art, each implementation disclosed herein can comprise, consist essentially of or consist of its particular stated element, step, or component. Thus, the terms "include" or "including" should be interpreted to recite: "comprise, consist of, or consist essentially of." The transition term "comprise" or "comprises" means has, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the implementation to the specified elements, steps, ingredients, or components and to those that do not materially affect the implementation. As used herein, the term "based on" is equivalent to "based at least partly on," unless otherwise specified.

Unless otherwise indicated, all numbers expressing quantities, properties, conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1% of the stated value.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing implementations (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate implementations of the disclosure and does not pose a limitation on the scope of the disclosure. No language in the specification should be construed as indicating any non-claimed element essential to the practice of implementations of the disclosure.

Groupings of alternative elements or implementations disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain implementations are described herein, including the best mode known to the inventors for carrying out implementations of the disclosure. Of course, variations on these described implementations will become apparent to those of ordinary skill in the art upon reading the foregoing description. The inventors expect skilled artisans to employ such variations as appropriate, and the inventors intend for implementations to be practiced otherwise than specifically described herein. Accordingly, the scope of this disclosure includes all modifications and equivalents of the subject matter recited in the claims appended hereto as permitted by applicable law. Moreover, any combination of the above-described elements in all possible variations thereof is encompassed by implementations of the disclosure unless otherwise indicated herein or otherwise clearly contradicted by context.

## Claims

1. A medical device, comprising:
an output device; and
a processor configured to:
receive data indicating an inflation pressure in a cuff inflating around a limb of a subject during an inflation time;
receive data indicating a deflation pressure in the cuff deflating around the limb of the subject during a deflation time;
generate a combined waveform by combining at least a portion of the data indicating the inflation pressure and at least a portion of the data indicating the deflation pressure;
determine a systolic blood pressure of the subject by analyzing the combined waveform;
determine a diastolic blood pressure of the subject by analyzing the combined waveform;
determine a mean arterial pressure of the subject by analyzing the combined waveform;
cause the output device to output an indication of the systolic blood pressure;
and cause the output device to output an indication of the diastolic blood pressure;
and cause the output device to output an indication of the mean arterial pressure.

2. The medical device of claim 1, wherein the processor is configured to generate the combined waveform by:
reversing the data indicative of the inflation pressure or the data indicative of the deflation pressure, resulting in alignable waveforms; and
aligning the alignable waveforms.

3. The medical device of claim 1, wherein:
the portion of the data indicative of the inflation pressure corresponds to a first pressure range bounded by a particular pressure,
the portion of the data indicative of the deflation pressure corresponds to a second pressure range bounded by the particular pressure, and
wherein the processor is configured to generate the combined waveform at least in part by adjoining the portion of the data indicative of the inflation pressure with the portion of the data indicative of the deflation pressure.

4. The medical device of claim 1, wherein the processor is configured to generate the combined waveform by cross-correlating, cross-referencing, cross-checking, superimposing, interpolating, or convolving the portion of the data indicative of the inflation pressure with the portion of the data indicative of the deflation pressure.

5. The medical device of claim 1, wherein the processor is configured to generate the combined waveform by determining an average of the portion of the data indicative of the inflation pressure and the portion of the data indicative of the deflation pressure.

6. The medical device of claim 1, wherein the processor is configured to generate the combined waveform by:
identifying, in the data indicative of the inflation pressure or the data indicative of the deflation pressure, one or more outlier values; and
omitting the one or more outlier values from the portion of the data indicative of the inflation pressure or the portion of the data indicative of the deflation pressure.

7. The medical device of claim 1, wherein the processor is further configured to:
determine, by analyzing the data indicative of the inflation pressure, one or more deflation pressures for use in generating the combined waveform; and
cause the cuff to deflate to the one or more deflation pressures in order to generate the data indicating the deflation pressure,
optionally wherein the medical device further comprises:
an input device configured to receive an operator input signal indicating activation of a selective deflation mode,
wherein the processor is configured to cause the cuff to deflate to the one or more deflation pressures in response to the activation of the selective deflation mode.

8. The medical device of claim 1, wherein the processor is further configured to:
receive additional inflation data indicating additional inflation pressures in the cuff inflating around the limb of the subject during an additional inflation time; or
receive additional deflation data indicating additional deflation pressures in the cuff deflating around the limb of the subject during an additional deflation time; and
discarding one or more of the additional inflation data or the additional deflation data in favor or using the portion of the data indicating the inflation pressure and the portion of the data indicating the deflation pressure to generate the combined waveform.

9. The medical device of claim 1, wherein the processor is further configured to use one or more features of the data indicating the inflation pressure to filter or gate the data indicating the deflation pressure.

10. A method, comprising:
receiving data indicating an inflation pressure in a cuff inflating around a limb of a subject during an inflation time;
receiving data indicating a deflation pressure in the cuff deflating around the limb of the subject during a deflation time;
generating a combined waveform by combining at least a portion of the data indicating the inflation pressure and at least a portion of the data indicating the deflation pressure;
determining a systolic blood pressure of the subject by analyzing the combined waveform;
determining a diastolic blood pressure of the subject by analyzing the combined waveform;
determining a mean arterial pressure of the subject by analyzing the combined waveform;
causing an output device to output an indication of the systolic blood pressure;
causing the output device to output an indication of the diastolic blood pressure; and
causing the output device to output an indication of the mean arterial pressure.

11. The method of claim 10, further comprising:
reversing the data indicative of the inflation pressure or the data indicative of the deflation pressure, resulting in alignable waveforms; and
aligning the alignable waveforms.

12. The method of claim 10, wherein:
the portion of the data indicative of the inflation pressure corresponds to a first pressure range bounded by a particular pressure,
the portion of the data indicative of the deflation pressure corresponds to a second pressure range bounded by the particular pressure, and
generating the combined waveform comprises adjoining the portion of the data indicative of the inflation pressure with the portion of the data indicative of the deflation pressure.

13. The method of claim 10, wherein generating the combined waveform comprises one or more of:
- cross-correlating, cross-referencing, cross-checking, superimposing, interpolating, or convolving the portion of the data indicative of the inflation pressure with the portion of the data indicative of the deflation pressure;
- determining an average of the portion of the data indicative of the inflation pressure and the portion of the data indicative of the deflation pressure;
- identifying, in the data indicative of the inflation pressure or the data indicative of the deflation pressure, one or more outlier values; and omitting the one or more outlier values from the portion of the data indicative of the inflation pressure or the portion of the data indicative of the deflation pressure.

14. The method of claim 10, further comprising:
determining, by analyzing the data indicative of the inflation pressure, one or more deflation pressures for use in generating the combined waveform; and
causing the cuff to deflate to the one or more deflation pressures in order to generate the data indicating the deflation pressure,
optionally wherein the method further comprises receiving an operator input signal indicating activation of a selective deflation mode, wherein causing the cuff to deflate to the one or more deflation pressures is in response to the activation of the selective deflation mode.

15. The method of claim 10, wherein generating the combined waveform comprises using one or more features of the data indicating the inflation pressure to filter or gate the data indicating the deflation pressure.
